# EUROPEAN PATENT APPLICATION

(11) **EP 2 769 722 A1**
(43) Date of publication of application: **27.08.2014**
(21) Application number: 13156342.1
(22) Date of filing: 22.02.2013
(51) Int. Cl.: A61K 31/505, A61K 31/506, A61P 31/18

(54) **Compounds for use in inhibiting HIV capsid assembly**

(71) Applicant: Ruprecht-Karls-Universität Heidelberg, 69117 Heidelberg (DE); Institute Of Organic Chemistry And Biochemistry As CR, V.V.I., 16610 Praha 6 (CZ)
(72) Inventor: Kräusslich, Hans-Georg, 68120 Heidelberg (DE); Sticht, Jana, 14195 Berlin (DE); Wildova, Marcela, 16500 Prag 6-Suchdol (CZ); Lux, Vanda, 40625 Düsseldorf (DE); Konvalinka, Jan, 16100 Prague 6 (CZ); Kotora, Martin, 153 00 Prague 5 (CZ); Grantz Sasková, Klára, 270 07 Mutejovice (CZ); Kozísek, Milan, 182 00 Prague 8 (CZ); Stepánek, Ondrej, 460 06 Liberec 6 (CZ); Parkan, Kamil, 130 00 Prague 3 (CZ); Machara, Ales, 169 00 Prague 6 (CZ)
(74) Representative: Dick, Alexander

(57) **Abstract**

The present invention relates a compound or a pharmaceutically acceptable salt or solvate thereof for use in inhibiting HIV capsid assembly, wherein the compound is a pyrimidine being at least substituted in two positions, preferably in 2 and 4 position or in 4 and 6 position, more preferably in 2 and 4 position.

## Description

The present invention relates to pyrimidine compounds and pharmaceutically acceptable salts or solvates thereof for use in inhibiting HIV capsid assembly, these pyrimidine compounds being at least substituted in two positions, preferably in 2 and 4 position or in 4 and 6 position, more preferably in 2 and 4 position.

The family of retroviruses comprises small enveloped virus particles (diameter ∼100 nm) carrying two copies of a single-stranded positive sense RNA genome. All retroviruses possess three major genes encoding for the polyproteins Gag (structural proteins), Pol (enzymes) and Env (surface glycoproteins). The most studied member of the retroviral family is human immunodeficiency virus type 1 (HIV-1 ), the causative agent of acquired immunodeficiency syndrome (AIDS) with approx. 33.4 million people infected worldwide and 2 million deaths due to AIDS in 2008 alone. In the past two decades highly active antiretroviral therapy (HAART) has been implemented. There are two types of HIV, HIV-1 and HIV-2, with HIV-1 accounting for the majority of infections. Currently there are available many anti-HIV-1 drugs targeting mainly viral enzymes (e.g. nucleoside and non-nucleoside reverse transcriptase inhibitors, protease inhibitors, integrase inhibitors) or the viral entry into the host cell (fusion inhibitors and attachment inhibitors). Available drugs are nowadays used preferentially in combinations; still the occurrence of drug-resistant variants is the main problem in HIV-1 treatment. Therefore any new target in the HIV-1 life cycle would be useful tool in anti-retroviral therapy.

The Gag polyprotein alone is necessary and sufficient to form virus like particles. Its multimerization is mainly driven by intermolecular interactions between the capsid (CA), spacer peptide 1 (sp1) and nucleocapsid (NC) region of neighboring Gag molecules. The Gag polyprotein precursor radially lines the inside of the viral membrane and forms the spherical immature core. Concomitant with, or shortly after release, Gag is processed into matrix, CA, sp1, NC, spacer peptide 2 (sp2) and protein of 6 kDa (p6). CA then builds up the conical core of the mature virion which encloses the viral genome. Only after this maturation process the virus become infectious.

In 2005 was published by Jana Sticht et al. (Nature structural and molecular biology 12:671-677, 2005) a 12-mer peptide, capsid assembly inhibitor (CAI), that binds the capsid (CA) domain of Gag and inhibits assembly of immature- and mature-like particles *in vitro.* CAI was identified by phage display screening among a group of peptides with similar sequences that bind to a single reactive site in CA. Its binding site was mapped to CA residues 169-191, with an additional contribution from the last helix of CA. This result was confirmed by a separate X-ray structure analysis at the 1.7-Å-resolution showing that CAI inserts into a conserved hydrophobic groove and alters the CA dimer interface (F. Ternois et al. Nature structural and molecular biology 12:678-682, 2005). The CAI binding site was suggested as a new target for antiviral development, and CAI was the first known inhibitor directed against assembly of immature HIV-1.

The detail interaction between C-terminal CA (C-CA) and CAI was further investigated by Vanda Bartonova (JBC 283:32024-32033, 2008) and in this study were identified the amino acid residues essential for C-CAICAI interaction. CA variants carrying mutations Y169A, L211A, or L211S had a reduced affinity for CAI and were unable to form mature-like particles *in vitro.* These mutations also blocked morphological conversion to mature virions in tissue culture and abolished infectivity. X-ray crystallographic analyses of the variant C-CA domains revealed that these alterations induced the same allosteric changes at the dimer interface observed in the C-CA/CAI complex.

Peptide inhibitors, however, have inherent drawbacks, as they usually have a short half-life and poor bioavailability. Accordingly it was shown that neither addition of the CAI peptide to virus-producing cells in culture nor peptide transfection caused any inhibition of HIV-1 release (J. Sticht et al, Nature structural and molecular biology 12:671-677, 2005). Thus, CAI cannot be directly used as an antiviral agent. Thus, there is a need for HIV capsid assembly inhibitors not having the drawbacks of the known capsid assembly inhibitors.

US 7,618,975 describes the use of 4-arylamino-quinazolines and of analogs thereof as activators of caspases and inducers of apoptosis. The compounds described in US 7,618,975 allow for induction of apoptosis, for inhibiting the assembly of tubulin and for inhibiting topoisomerase II. However, the document does not disclose 2,4 or 4,6-substituted pyrimidines can be used for inhibiting HIV capsid assembly.

Tian et al. (Bioorg. Med. Chem- Lett. 19 (2009) 2162-2167) discloses the use of acylhydrazone derivatives for inhibiting HIV-1 capsid assembly in vitro.

Li et al. (Bioorganic & Medicinal Chemistry 17 (2009) 3177-3188) discloses that thiourea compounds for inhibiting HIV-1 capsid assembly in vitro.

However, there is still a need for effective HIV capsid assembly inhibitors. Thus, the present invention relates to a compound or pharmaceutically acceptable salts or solvates thereof for use in inhibiting HIV capsid assembly, the compound being a pyrimidine which is at least substituted in 2 and 4 or 4 and 6 position.

Further, the present invention relates to a pharmaceutical composition for inhibiting HIV capsid assembly comprising the compound, as described above, in a therapeutically affective amount, and the use of a compound, as described above, for the manufacture of a medicament for inhibiting HIV capsid assembly.

Further, the present invention relates to a method of treating a patient suffering from HIV comprising administering a therapeutically effective amount of the compound as defined above, as well as the use of this compound for treating a patient suffering from HIV.

The term "pyrimidine substituted at least in 2 and 4 position" as used in the context of the present invention is denoted to mean that - besides the substitutions in 2 and 4 position, the shown structure below may be further suitably substituted.

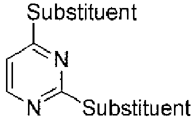

The term "pyrimidine substituted at least in 4 and 6 position" as used in the context of the present invention is denoted to mean that - besides the substitutions in 4 and 6 position, the shown structure below may be further suitably substituted.

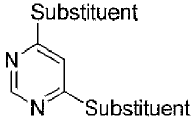

Preferably, the compound, described above, is substituted in 2 and 4 position, thus having the structure

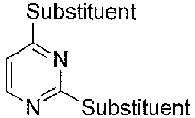

More preferably the compound has a structure according 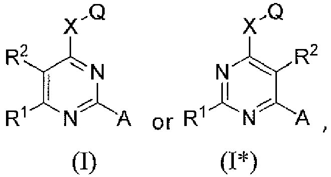 in particular according to formula (I) 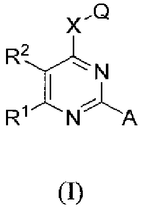 wherein A is selected from the group consisting of aryl, heteroaryl and heterocycloalkyl group, X is selected from the group consisting of O, N(R³) and S, and wherein R³ is selected from the group consisting of H, alkyl, cycloalkyl, aryl, heteroaryl and heterocycloalkyl, Q is selected from the group consisting of alkyl, cycloalkyl, aryl, heteroaryl and heterocycloalkyl, and wherein R¹ and R², are, independently of each other, selected from the group consisting of H, D, alkyl, cycloalkyl, cycloalkenyl, heterocycloalkyl, aryl, heteroaryl, alkenyl, alkoxy and halides.

Within the meaning of the present invention, the term "alkyl" relates to non-branched alkyl residues and branched alkyl residues, as well as residues comprising one or more heteroatoms or functional groups, such as, by way of example, -O-, -S-, -NH-, -NH-C(=O)-, -C(=O)-NH-, and the like. The term also encompasses alkyl groups which are further substituted by one or more suitable substituent. Preferably, the alkyl groups according to the invention have from 1 to 20 carbon atoms, more preferably from 1 to 10 carbon atoms. Such alkyl groups are hereinunder also referred to as C1-20alkyl and C1-10alkyl, respectively.

The term "substituted" as used in the context of the present invention preferably refers to organic groups in which a hydrogen group, in any position, is replaced by one or more substituents, preferably by 1, 2, 3, 4, 5 or 6 substituents, more preferably by 1, 2, or 3 substituents. If two or more substituents are present, each substituent may be the same or may be different from the at least one other substituent. There are in general no limitations as to the substituent. The substituents may be, for example, selected from the group consisting of aryl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamide, trifluoromethyl, cyano, azido, cycloalkyl such as e.g. cyclopentyl or cyclohexyl, heterocycloalkyl such as e.g. morpholino, piperazinyl or piperidinyl, alkylaryl, arylalkyl and heteroaryl. Preferred substituents of such organic residues are, for example, halogens, such as in particular fluorine or iodine, amino groups, hydroxyl groups, carbonyl groups, thiol groups and carboxyl groups.

Within the meaning of the present invention, the term "cyclic alkyl" or "cycloalkyl" relates to optionally suitably substituted to 8-membered single-ring alkyl groups as well as optionally suitably substituted multicyclic alkyl residues preferably having up to 20, more preferably up to 10 carbon atoms. Cycloalkyl groups residues having up to 20, preferably up to 10 carbon atoms, also referred to as C1-20cycloalkyl and C1-10cycloalkyl, respectively. By way of example, the following cycloalkyl groups are mentioned: cyclopentyl, cyclohexyl, cycloheptyl cyclopropylmethyl, cyclopentylmethyl, cyclohexylmethyl, cyclohexylcyclopentylmethyl, dicyclohexylmethyl, 1-cyclopentylethyl, 1-cyclohexylethyl, 2-cyclopropylethyl, 2-cyclopentylethyl, 2-cyclohexylethyl, 2-cycloheptylethyl, 1-cyclohexyl-1-methylethyl, 1-cyclohexylpropyl, 2-cyclopentylpropyl, 3-cyclobutylpropyl, 3-cyclopentylpropyl, 3-cyclohexylpropyl, 3-cycloheptylpropyl, 1-cyclopropyl-1methylpropyl, 1-cyclohexyl-2-methylpropyl, 1-cyclopentyl butyl, 1-cyclohexylbutyl, 3-cyclohexylbutyl, 4-cyclopropylputyl, 4-cyclobutylbutyl, 4-cyclopentylbutyl, 1-cyclohexyl 1-methylbutyl, 1-cyclopentyl-2-ethylbutyl, 1-cyclohexyl-3methylbutyl, 1-cyclopentylpentyl, 1-cyclohexylpentyl, 1-cyclohexylmethylpentyl, 2-cyclohexylpentyl, 2-cyclohexylmethylpentyl, 3-cyclopentylpentyl, 1-cyclohexyl-4methylpentyl, 5-cyclopentylpentyl, 1-cyclopentylhexyl, 1-cyclohexylhexyl, 1-cyclopentylmethylhexyl, 2-cyclopentylhexyl, 2-cyclopropylethylhexyl, 3-cyclopentylhexyl, 1-cyclohexylheptyl, 1 -cyclopentyl-1-methylheptyl, 1-cyclohexyl-1,6-dimethylheptyl, 1-cycloheptyloctyl, 2-cyclopentyloctyl, 3-cyclohexyloctyl, 2-cyclopentylmethyloctyl, 1-cyclopentylnonyl, 1-cyclohexylnonyl, 3-cyclopropylnonyl, 1-cyclopentyldecyl, 1-cyclohexylundecyl, 1-cyclopentyl tridecyl, 2-cyclohexyltridecyl, and the like.

Within the meaning of the present invention, the term "heterocycloalkyl" relates to optionally suitably substituted 3 to 8-membered single-ring alkyl groups as well as optionally suitably substituted multicyclic alkyl residues having preferably up to 20, more preferably up to 10 carbon atoms, wherein the cyclic alkyl groups comprise one or more, preferably from 1 to 4 such as 1, 2, 3 or 4, heteroatoms, wherein in case the cycloalkyl residue comprises more than 1 heteroatom, the heteroatoms may be the same or different. Heterocycloalkyl groups residues having up to 20, preferably up to 10 carbon atoms, also referred to as C1-20heterocycloalkyl and C1-10heterocyclocloalkyl, respectively.

Preferably, the heteroaroms are selected from the group consisting of O, S and N.

The term "alkyl", "cycloalkyl", "cyclic alkyl" and "heterocycloalkyl", also encompasses groups which are further substituted by one or more suitable substituent.

Within the meaning of the present invention, the term "aryl" refers to, but is not limited to, optionally suitably substituted 5- and 6-membered single-ring aromatic groups as well as optionally suitably substituted aryl groups. The term "aryl" thus includes, for example, optionally substituted phenyl groups or optionally suitably substituted naphthyl groups. Aryl groups can also be annealed with alicyclic or heterocycloalkyl rings that are not aromatic so as to form a polycycle, e.g. benzodioxolyl or tetraline. The term "aryl" further includes aromatic groups which linked via a single bond to further aromatic groups so as to form, e.g., biphenyl groups.

The term "heteroaryl" as used within the meaning of the present invention includes optionally suitably substituted 5- and 6-membered single-ring aromatic groups as well as substituted or unsubstituted multicyclic aryl groups, for example bicyclic or tricyclic aryl groups, comprising one or more, preferably from 1 to 4 such as 1, 2, 3 or 4, heteroatoms, wherein in case the aryl residue comprises more than 1 heteroatom, the heteroatoms may be the same or different. Such heteroaryl groups including from 1 to 4 heteroatoms are, for example, benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, benztriazyl, isoindolyl, benzofuranyl, purinyl, deazapurinyl, pteridinyl, or indolizinyl.

The term "substituted aryl" and the term "substituted heteroaryl" as used in the context of the present invention describes moieties having substituents replacing a hydrogen on one or more atoms, e.g. C or N, of an aryl or heteroaryl moiety. Again, there are in general no limitations as to the substituent. The substituents may be, for example, selected from the group consisting of alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, cycloalkyl such as e.g. cyclopentyl or cyclohexyl, heterocycloalkyl such as e.g. morpholino, piperazinyl or piperidinyl, alkylaryl, arylalkyl and heteroaryl.

The term "alkenyl" as used in the context of the present invention refers to unsaturated alkyl groups having at least one double bond. The term also encompasses alkenyl groups which are substituted by one or more suitable substituent.

The term "cycloalkenyl" as used in the context of the present invention refers to unsaturated cycloalkyl groups having at least one double bond. The term also encompasses alkenyl groups which are substituted by one or more suitable substituent. By way of example, the following groups are mentiond: cyclopentenyl, cyclohexenyl, cycloheptenyl, cyclooctenyl, cyclopentadienyl, cyclohexadienyl, cycloheptadienyl, cyclooctadienyl, and the like. Preferred are those with 5-7 carbon atoms, including cyclopentenyl, cyclohexenyl, and cycloheptenyl.

The term "alkynyl" refers to unsaturated alkyl groups having at least one triple bond. The term also encompasses alkynyl groups which are substituted by one or more suitable substituent.

The term "alkoxy", as used herein, refers to an alkyl residue being linked via an oxygen group to the respective parent molecule, thus to a residue having the structure -O-alkyl, wherein this term is meant to also encompass groups in which the alkyl group bears further substituents, as mentioned above.

### The group X

As described above, X is selected from the group consisting of O, N(R³) and S, and wherein R³ is selected from the group consisting of H, alkyl, cycloalkyl, aryl, heteroaryl and heterocycloalkyl. Preferably R³ is H or alkyl, more preferably H, methyl or ethyl, more preferably H or methyl.

Thus, according to a preferred embodiment X is selected from the group consisting of O, N(CH₃), NH and S, more preferably X is N(CH₃) or NH, in particular NH:

Thus, the present invention also relates to a compound, as described, having one of the following structures: 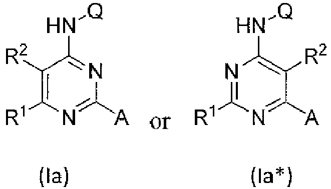 preferably having the structure (Ia)

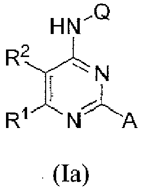

### The residue A

As described above, A is selected from the group consisting of aryl, heteroaryl and heterocycloalkyl. It is to be understood that this definition encompasses substituted as well as unsubstituted aryl, heteroaryl and heterocycloalkyl groups. Preferably A is a, substituted or unsubstituted, aryl or heteroaryl group.

In particular A is a, substituted or unsubstituted, heteroaryl group, preferably a, substituted or unsubstituted, heteroaryl group comprising at least one N, in particular A is selected from the group consisting of, substituted or unsubstituted, pyrrolyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, quinolinyl, isoquinolinyl, benztriazyl, isoindolyl, purinyl, deazapurinyl, pteridinyl, or indolizinyl.

More preferably A is a, substituted or unsubstituted, pyridine group or pyridazin group. According to a preferred embodiment of the invention, A has the structure 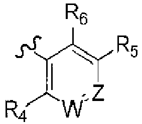 wherein W is N or C(R^{W}), and wherein Z is N or C(R^{Z}), and wherein R^{W} and R^{Z} are, independently of each other selected from the group consisting of H, alkyl, -O-alkyl, and halide, and wherein R⁴, R⁵ and R⁶, are, independently of each other, selected from the group consisting of -H, -alkyl, -O-alkyl, -halides.

More preferably, in case W is N, Z is C(R^{Z}), and in case Z is N, W is C(R^{W}), thus, A is preferably a pyridine group.

Thus, the present invention also relates to a compound, as described above, wherein A has the structure

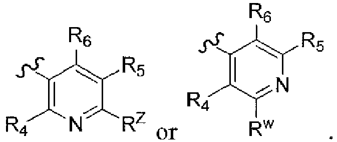

As regards R⁴, R⁵, R⁶, R^{W} and R^{Z}, these residues are, preferably, independently of each other selected from the group consisting of H, methyl, Br and Cl,

Thus, by way of example, the following preferred residues A are mentioned:

**Table 1: Preferred residues A**

| R⁴ | R⁵ | R⁶ | W | Z |
|---|---|---|---|---|
| H, methyl, Br or Cl | H | H | N | C-H |
| H, methyl, Br or Cl | H | H | C-H | N |
| H | methyl, Br or Cl | H | N | C-H |
| H | methyl, Br or Cl | H | C-H | N |
| H | H | methyl, Br or Cl | N | C-H |
| H | H | methyl, Br or Cl | C-H | N |

Preferably, A is selected from the group consisting of

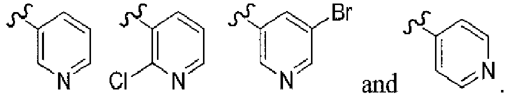

Thus, the present invention also relates to a compound, as described, having the structure (I) or (I*), more preferably the structure (I), more preferably the structure (Ia) or (Ia*), and even more the structure (Ia) 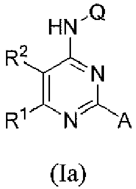 wherein A is selected from the group consisting of

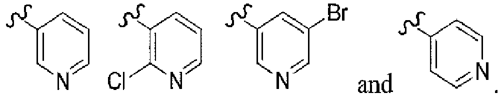

Thus, the compound more preferably has a structure selected from the group consisting of

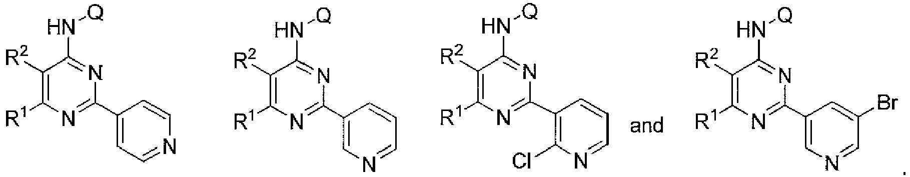

### The residue Q

As described above, Q is selected from the group consisting of alkyl, cycloalkyl, aryl, heteroaryl and heterocycloalkyl.

Preferably Q is selected from the group consisting of C1-10alkyl, C1-10alkoxy, C3-10cycloalkyl, C3-10heterocycloalkyl, C6-10aryl, C1-4alkoxy-C1-10alkyl and C5-10heteroaryl. In case Q is an aryl group, Q is preferably phenyl, or a benzocyclopentadienyl group, or a benzocyclopentyl group. In case Q is an alkyl group, Q is preferably selected from the group consisting of methyl, ethyl, propyl, -CF₃, -CHF₂, -CH₂F, -CH₂-CF₃, -CH₂-CHF₂, -CH₂-CH₂F, mono/di/tri...polyhaloalkyls and mono/diltri...polyhalocycloalkyls (like 2-fluoroethyl). In case Q is an heteroaryl group, Q is preferably selected from the group consisting of indolyl, benzimidazolyl, pyridinyl, benztriazyl pyrimidinyl and pyrazinyl. In case Q is an cycloalkyl group, Q is preferably selected from the group consisting of cyclohxane, cyclopentane. In case Q is an cycloheteroalkyl group, Q is preferably selected from the group consisting of morpholino, piperazinyl, piperidinyl tetrahydrofuran, pyrrolidin, pyrrolidin-2-on. t is to be understood that all above mentioned residues mentioned may be substituted or unsubstituted.

Thus according to a preferred embodiment, Q is selected from the group consisting phenyl, benzocyclopentadienyl group, benzocyclopentyl group, methyl, ethyl, propyl, -CF₃, -CHF₂, - CH₂F, -CH₂-CF₃, -CH₂-CHF₂, -CH₂-CH₂F, indolyl, benzimidazolyl, pyridinyl, benztriazyl pyrimidinyl, pyrazinyl, cyclohexane, cyclopentane, morpholino, piperazinyl, piperidinyl tetrahydrofuran, pyrrolidin, pyrrolidin-2-on, wherein all residues mentioned (except for CF₃, - CHF₂, -CH₂F, -CH₂-CF₃, -CH₂-CHF₂, -CH₂-CH₂F) may be substituted or unsubstituted.

According to a one preferred embodiment of the invention, Q is selected from the group consisting of an C1-10alkyl group, which is optionally substituted with at least one fluorine group.

According to an alternative preferred embodiment of the present invention, Q is an aryl group or heteroaryl group. It is to be understood that this definition includes substituted as well as unsubstituted aryl groups as well as substituted and unsubstituted heteroaryl groups.

Preferably has one of the following structures: 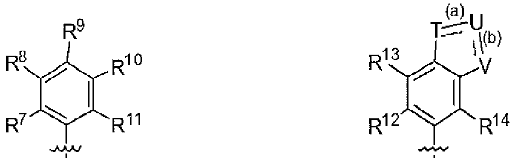 wherein R⁷, R⁸, R⁹, R¹⁰, R¹¹, R¹², R¹³ and R¹⁴ are, independently of each other, selected from the group consisting of -H, -alkyl, -halides, alkynyl, alkenyl, -NO₂, -CN, -C(=Y)-R¹⁵, -C(=Y)-H, - C(=Y)-Y¹-R¹⁵, -C(=Y)-OH, , -Y-R¹⁵, -NH₂, -NHR¹⁵,-NH -C(=Y)-R¹⁵, -CF₃, -S(O)R¹⁵,-SO₂R¹⁵, -P(O)R¹⁵R¹⁶, -P(O)(OR¹⁵)R¹⁶ and -P(O)(OR¹⁵)(OR¹⁶), wherein Y is O, S or NH, and wherein Y¹ is O, S or NR*, wherein R* is H or alkyl, and wherein R¹⁵ and R¹⁶ are, independently of each other, selected from the group consisting of -H, -alkyl, -alkenyl, -heterocycloalkyl, aryl and heteroaryl,
and wherein
wherein T is -C- R^{T}, N or N-Y^{T}, with Y^{T} being H or alkyl
wherein U is -C-R^{U}, N or N-Y^{U}, with Y^{U} being H or alkyl
wherein V is -C-R^{V}, N or N-Y^{Y}, withy Y^{V} being H or alkyl
the bond (a) is a single or double bond,
the bond (b) is a single or double bond,
with eth proviso that one of (a) and (b) is a double bond,
wherein R^{T}, R^{U}, and R^{V} are, independently of each other, H or -alkyl.

Thus, the present invention also describes a compound for inhibiting HIV capsid assembly, as described above, the conjugate having the structure (I) or (I*), more preferably the structure (I), more preferably the structure (Ia) or (Ia*), and even more the structure (Ia), wherein Q has the structure: 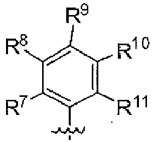 wherein R⁷, R⁸, R⁹, R¹⁰ and R¹¹ are, independently of each other, selected from the group consisting of -H, -alkyl, -halides, alkynyl, alkenyl, -NO₂, -CN, -C(=Y)-R¹⁵, -C(=Y)-H, -C(=Y)-Y-R¹⁵, -C(=Y)-OH, , , -Y-R¹⁵, -NH₂, -NHR¹⁵ -NH -C(=Y)-R¹⁵ -CF₃ -S(O)R¹⁵ -SO₂R¹⁵, - P(O)R¹⁵R¹⁶, -P(O)(OR¹⁵)R¹⁶ and -P(O)(OR¹⁵)(OR¹⁶), wherein Y is O, S or NH, and wherein Y¹ is O, S or NR*, wherein R* is H or alkyl, and wherein R¹⁵ and R¹⁶ are, independently of each other, selected from the group consisting of -H, -alkyl, -alkenyl, -heterocycloalkyl, aryl and heteroaryl. It is to be understood that all above mentioned residues denoted as alkyl, alkoxy, alkynyl, alkenyl may be substituted or unsubstituted. Further, in case more than one group R¹⁵ or R¹⁶ is present, the respective groups may be the same or may differ from each other. In case any one of R⁷, R⁸, R⁹, R¹⁰ and R¹¹ is an alkyl group, this alkyl group is preferably selected from the group consisting of methyl, ethyl, propyl, -CF₃, -CHF₂, -CH₂F, -CH₂-CF₃, -CH₂-CHF₂, -CH₂-CH₂F

According to a preferred embodiment, R⁷, R⁸, R⁹, R¹⁰ and R¹¹ are, independently of each other, selected from the group consisting of -C(=Y)-R¹⁵, -C(=Y)-H, -C(=Y)-Y¹-R¹⁵, -C(=Y)-OH, -OR¹⁵, -NH-C(=Y)-R¹⁵, -C(=Y)-NH-R¹⁵, -CF₃, -Cl and F, more preferably, , independently of each other, selected from the group consisting of -C(=O)-alkyl, -C(=O)-H, -C(=O)-OH, -C(=O)-O-alkyl, -NH-C(=Y)-OH, , -NH-C(=Y)-aryl, -O-alkyl -NH-alkyl, CF₃ and -F., more preferably selected from the group consisting of -C(=O)-ethyl, -C(=O)-methyl -C(₌O)-NH-CH₂-CH₂-F - C(=O)-H, -C(=O)-OH, -C(=O)-O-ethyl, -C(=O)-O-methyl -NH-C(=O)-OH, -O-methyl, -O-CH₂-CH₂-N(CH₂CH₃)₂, -NH-C(=Y)-(3-trifluoromethylphenyl) -CF₃,-Cl and -F

**Table 2: Preferred residues Q**

| R⁷ | R⁸ | R⁹ | R¹⁰ | R¹¹ |
|---|---|---|---|---|
| -C(=O)-ethyl, -C(=O)-methyl -C(=O)-NH-CH₂-CH₂-F -C(=O)-H, -C(=O)-H, -C(=O)-O-ethyl, -C(=O)-O-methyl -NH-C(=O)-OH, -O-methyl, -NH-C(-Y)-(3-trifluoromethylphen Y1) -O-CH₂-CH₂-N(CH₂CH₃)₂, -CF₃, -Cl or -F | H | H | H | H |
| H | -C(=O)-ethyl, -C(=O)-methyl -C(=O)-MH-CH₂-CH₂-F -C(=O)-H, -C(=O)-H, -C(=O)-O-ethyl, -C(=O)-O-methyl -NH-C(=O)-OH, -O-methyl, -NH-C(=Y)-(3-trifluoroznethylphen yl) -O-CH₂-CH₂-N(CH₂CH₃)₂, -CF₃, -Cl or -F | H | H | H |
| H | H | -C(=O)-ethyl, -C(=O)-methyl -C(=O)-NH-CH₂-CH₂-F -C(=O)-H, -C(=O)-H, -C(=O)-O-ethyl, -C(=O)-O-methyl -NH-C(=O)-OH, -O-methyl, -NH-C(-Y)-(3-trifluoromethylphen yl) -O-CH₂-CH₂-N(CH₂CH₃)₂, -CF₃, -Cl or -F | H | H |
| H | H | H | -C(=O)-ethyl, -C(=O)-methyl -C(=O)-NH-CH₂-CH₂-F -C(=O)-H, -C(=O)-H, -C(=O)-O-ethyl, -C(=O)-O-methyl -NH-C(=O)-OH, -O-methyl, -NH-C(=Y)-(3-trifluoromethylphen yl) -O-CH₂CH₂-N(CH₂CH₃)₂, -CF₃, -Cl or -F | H |
| H | H | H | H | -C(=O)-ethyl, -C(=O)-methyl -C(=O)-NH-CH₂-CH₂-F -C(=O)-H, -C(=O)-H, -C(=O)-O-ethyl, -C(=O)-O-methyl -NH-C(=O)-OH, -O-methyl, -NH-C(=Y)-(3-trifluoromethylphenyl) -O-CH₂-CH₂-N(CH₂CH₃)₂, -CF₃, -Cl or -F |
| H | -O-methyl | -O-methyl | -O-metl,Yl | H |
| H | -F | -F | H or -F | H |
| H | -Cl | C(=O)-ethyl, -C(=O)-methyl -C(=O)-OH, -NH-C(=Y)-(3-trifluoromethylphen yl), | H | H |
| H | H | C(=O)-ethyl, -C(=O)-methyl -C(=O)-OH, -NH-C(=Y)-(3-trifluoromethylphen yl), -C(=O)-NH-CH₂-CH₂-F | F | H |

More preferably Q is selected from the group consisting

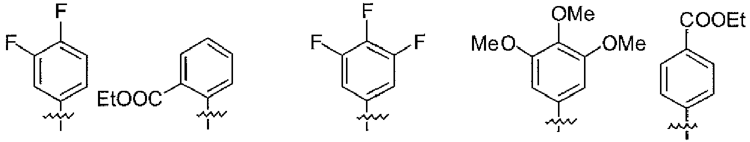

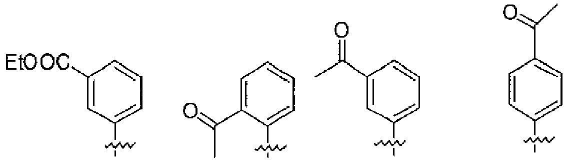

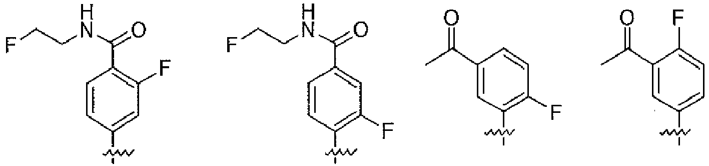

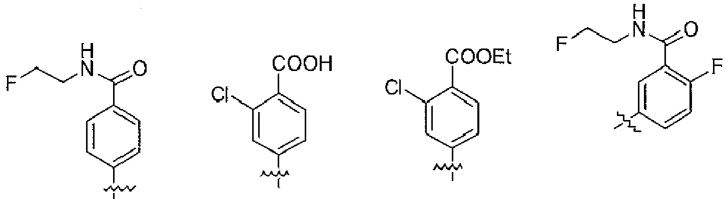

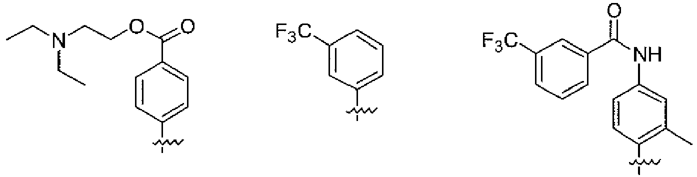

Thus, the present invention also describes a compound for inhibiting HIV capsid assembly, as described above, the conjugate having the structure (I) or (I*), more preferably the structure (I), more preferably the structure (Ia) or (Ia*), and even more the structure (Ia), wherein Q has the structure 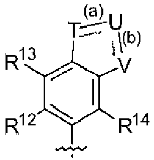 wherein R¹², R³³ and R¹⁴ are, independently of each other, selected from the group consisting of-H, -alkyl, -halides, alkynyl, alkenyl, -NO₂, -CN, -C(=Y)-R¹⁵, -C(=Y)-H, -C(=Y)-Y¹-R¹⁵, -C(=Y)-OH,, -Y-R¹⁵, -NH₂, -NHR¹⁵,-NH -C(-Y)-R¹⁵, -CF₃, -S(O)R¹⁵, -SO₂R¹⁵, -P(O)R¹⁵R¹⁶, - P(O)(OR¹⁵)R¹⁶ and -P(O)(OR¹⁵)(OR¹⁶), wherein Y is O, S or NH, and wherein Y¹ is O, S or NR*, wherein R* is H or alkyl, and wherein R¹⁵ and R¹⁶ are, independently of each other, selected from the group consisting of-H, -alkyl, -alkenyl, -heterocycloalkyl, aryl and heteroaryl, and wherein
wherein T is -C-R^{T}, N or N-Y^{T}, with Y^{T} being H or alkyl
wherein U is -C-R^{U}, N or N-Y^{U}, with Y^{U} being H or alkyl
wherein V is -C-R^{V}, N or N-Y^{Y}, with Y^{V} being H or alkyl
the bond (a) is a single or double bond,
the bond (b) is a single or double bond,
with eth proviso that one of (a) and (b) is a double bond,
wherein R^{T}, R^{U}, and R^{V} are, independently of each other, H or -alkyl.

Preferably, R¹² , R¹³ and R¹⁴ , are independently of each other selected from the group consisting of -H, -Cl -10alkyl, -F, -I, C1-10alkoxy, C2-10alkynyl, C2-10alkenyl, -NO₂ -C(-O)-C1-10alkyl, -C(=O)-H, -C(=O)-O-Cl-10alkyl, -C(=O)-OH and -O-C1-10alkyl, more preferably R¹², R¹³ and R¹⁴, are independently of each other selected from the group consisting of -H, -CH3, -CH2-CH3, CF₃, -CHF₂, -CH₂F, -CH₂-CF₃, -CH₂-CHF₂ and -CH₂-CH₂F, -F, -I, methoxy, ethoxy, -NO₂, - C(=O)-methyl, C(=O)- ethyl, -C(=O)-H, -C(=O)-O-methyl, -C(=O)-O-ethyl and -C(=O)-OH.

In particular, Q is selected from the group consisting of

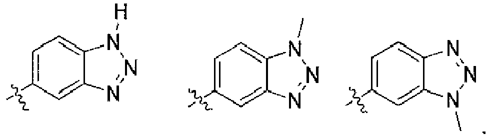

Thus, the present invention also describes a compound for inhibiting HIV capsid assembly, as described above, the conjugate having the structure (I) or (I*), more preferably the structure (I), more preferably the structure (Ia) or (Ia*), and even more the structure (Ia), more preferably one of the following structures 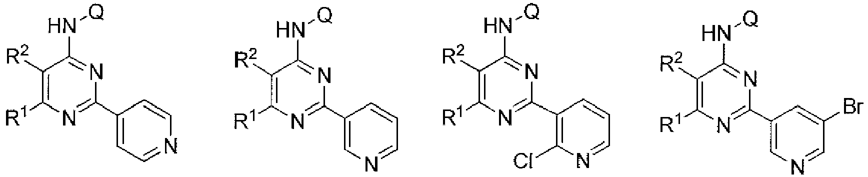 wherein Q is selected from the group consisting of

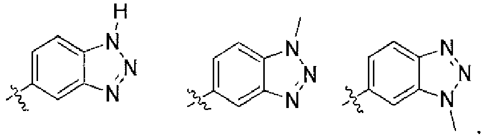

Further, the present invention also describes a compound for inhibiting HIV capsid assembly, as described above, the conjugate having a structure according to formula (I) or (I*), more preferably according to formula (I), more preferably according to formula (Ia) or (Ia*), and even more preferably according to formula (Ia), wherein Q is -alkyl-F, preferably -CH₂-CH₂-F.

### The functional groups R¹ and R²

As described above, R¹ and R², are, independently of each other, selected from the group consisting of H, D, -alkyl, cycloalkyl, cycloalkenyl, heterocycloalkyl, aryl, heteroaryl, alkenyl, alkoxy and halides.

In case any one of R¹ or R² is C1-10alkyl , the following C₁-₁₀alkyl groups are preferred: methy, trifluoromethyl, ethyl, propyl, butyl, pentyl, hexyl or octyl.

In case any one of R¹ or R² is C3-10cycloalkyl, the following preferred C3-10cycloalkyl groups are mentioned, by way of example: cyclopentyl, cyclohexyl, cycloheptyl cyclopropylmethyl, cyclopentylmethyl, cyclohexylmethyl, cyclohexylcyclopentylmethyl, dicyclohexylmethyl, 1 - cyclopentylethyl, 1-cyclohexylethyl, 2-cyclopropylethyl, 2- cyclopentylethyl, 2-cyclohexylethyl, 2-cycloheptylethyl, 1-cyclohexyl-1-methylethyl, 1-cyclohexylpropyl, 2-cyclopentylpropyl, 3-cyclobutylpropyl, 3-cyclopentylpropyl, 3- cyclohexylpropyl, 3-cycloheptylpropyl, 1-cyclopropyl-lmethylpropyl, 1-cyclohexyl-2-methylpropyl, 1-cyclopentyl butyl, 1-cyclohexylbutyl, 3-cyclohexylbutyl, 4-cyclopropylputyl, 4-cyclobutylbutyl, 4-cyclopentylbutyl, 1-cyclohexyl 1-methylbutyl, 1-cyclopentyl-2-ethylhutyl, 1-cyclohexyl-3methylbutyl, 1-cyclopentylpentyl, 1-cyclohexylpentyl, 1-cyclohexyhnethylpentyl, 2-cyclohexylpentyl, 2-cyclohexylmethylpentyl, 3-cyclopentylpentyl, 1-cyclohexyl-4methylpentyl, 5-cyclopentylpentyl, 1-cyclopentylhexyl, 1-cyclohexylhexyl, 1-cyclopentylmethylhexyl, 2-cyclopentylhexyl, 2-cyclopropylethylhexyl, 3-cyclopentylhexyl, 1-cyclohexylheptyl, 1 -cyclopentyl-1-methylheptyl, 1-cyclohexyl-1,6-dimethylheptyl, 1-cycloheptyloctyl, 2-cyclopentyloctyl, 3-cyclohexyloctyl, 2-cyclopentylmethyloctyl, 1-cyclopentylnonyl, 1-cyclohexylnonyl, 3-cyclopropylnonyl, 1-cyclopentyldecyl, 1-cyclohexylundecyl, 1-cyclopentyl tridecyl, 2-cyclohexyltridecyl, and the like

In case any one of R¹ or R² is C3-10heterocycloalkyl the following preferred C3-10heterocycloalkyl groups are mentioned by way of example: morpholino, piperazinyl, piperidinyl tetrahydrofuran, pyrrolidin, pyrrolidin-2-on.

In case any one of R¹ or R² is C3-10cycloalkenyl, the following preferred C3-10cycloalkenyl groups are mentioned by way of example: cyclopentenyl, cyclohexenyl, and cycloheptenyl. Further, the following preferred C2-20alkenyl groups are mentioned: ethenyl, propenyl, butenyl, pentenyl.

Further, the following preferred C6-10aryl groups are mentioned: phenyl (including substituted phenyl groups), benzyl (including substituted benzyl groups), naphthyl, anthryl, phenanthryl, biphenyl, and the like.

Further, the following preferred C5-10heteroaryl are mentioned: benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, indolyl, benzofuranyl, purinyl, deazapurinyl, or indolizinyl.

Most preferably, R¹ or are selected from the group consisting of C1-20alkyl, C3-10cycloalkyl, C3-10heterocycloalkyl, C3-10cycloalkenyl, C2-20alkenyl, C6-10aryl, C5-10heteroaryl, C(O)R^{1#}, C(O)OR^{1#}, -H, -D, -Cl -10alkoxy, and -F, wherein R^{1#} is C₁₋₁₀alkyl.

According to a particularly preferred embodiment, R¹ and R², are independently of each other, methyl, -CF₃ or H, more preferably R¹ is selected from the group consisting of methyl, -CF₃, and H and R² is -H.

Thus, the present invention also relates to a compound, as described above, having the structure: 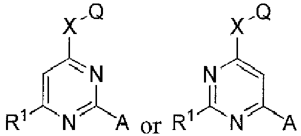 more preferably being selected from the group consisting of: 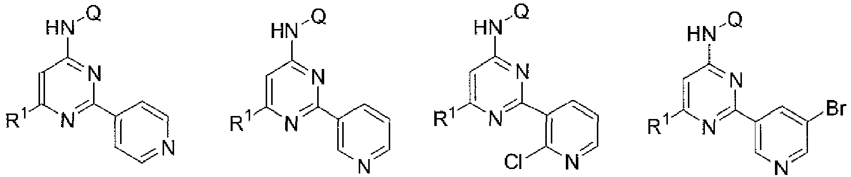 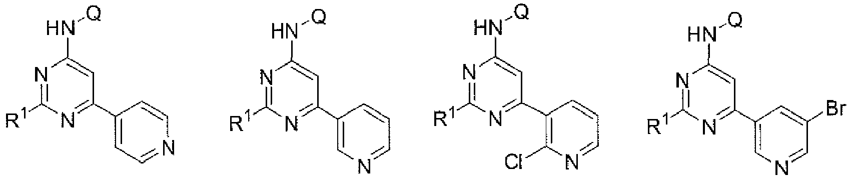 more preferably being selected from the group consisting of: 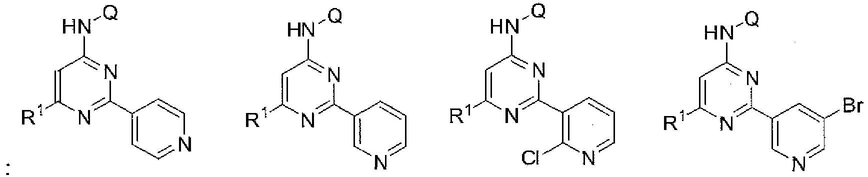 preferably R¹ is selected from the group consisting of methyl, -CF₃ and H. According to one preferred embodiment, both R¹ and R² are H.

According to a particularly preferred embodiment, the compound, as described above, has one of the following structures:

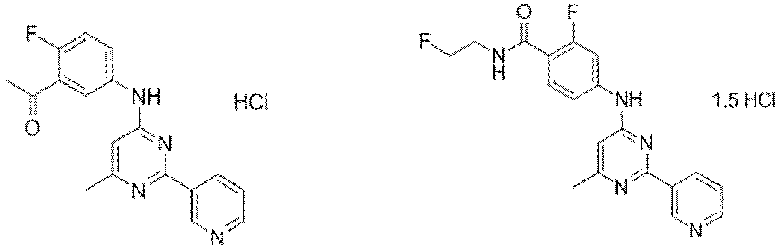

In a preferred embodiment of the present invention, the compound of the present invention is combined with at least one further antiretroviral agent. Preferred further antiretroviral agents are disclosed elsewhere herein. Thus, the present invention also envisage a composition comprising the compound of the present invention and at least one further antiretroviral agent.

### Pharmaceutically acceptable salt

As described above, the compounds of the present invention can be formulated as pharmaceutically acceptable salt or solvate.

The term "pharmaceutically acceptable" refers to those compounds or salts and solvates thereof as well as pharmaceutical, compositions, and/or dosage forms which are, within the scope of sound medical judgment, suitable for treatment of human beings and animals without excessive toxicity, irritation, allergic response, or other problem complications commensurate with a reasonable benefit/ risk ratio.

Typical pharmaceutically acceptable salts include those salts prepared by reaction of the compounds of the present invention with a pharmaceutically acceptable mineral or organic acid or an organic or inorganic base. Such salts are known as acid addition and base addition salts. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid. Salts of amine groups may also comprise quaternary ammonium salts in which the amino nitrogen carries a suitable organic group such as an alkyl, alkenyl, alkynyl, or aralkyl moiety. Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. It should be recognized that the particular counter ion forming a part of any salt of this invention is usually not of a critical nature, so long as the salt as a whole is pharmacologically acceptable and as long as the counter ion does not contribute undesired qualities to the salt as a whole.

The term acceptable solvate encompasses also pharmaceutically acceptable solvates of the compounds of the invention, wherein the compound combines with a solvent such as water, methanol, ethanol or acetonitrile to form a pharmaceutically acceptable solvate such as the corresponding hydrate, methanolate, ethanolate or acetonitrilate.

### Pharmaceutical Composition

As described above, the present invention also relates to a pharmaceutical composition comprising in a therapeutically effective amount the compound of the invention..

The term "therapeutically effective amount", as used herein, preferably refers to an amount of the compound, as defined herein, that effectively inhibits the HIV capsid assembly.

As far as the pharmaceutical compositions according to the present invention, are concerned, the conjugate may be used in combination with a pharmaceutical excipient. Generally, the compound will be in a solid form which can be combined with a suitable pharmaceutical excipient that can be in either solid or liquid form. As excipients, carbohydrates, inorganic salts, antimicrobial agents, antioxidants, surfactants, buffers, acids, bases, and combinations thereof may be mentioned. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, sorbitol (glucitol), pyranosyl sorbitol, myoinositol, and the like. The excipient may also include an inorganic salt or buffer such as citric acid, sodium chloride, potassium chloride, sodium sulfate, potassium nitrate, sodium phosphate monobasic, sodium phosphate dibasic, and combinations thereof. The pharmaceutical composition according to the present invention may also comprise an antimicrobial agent for preventing or determining microbial growth, such as, e.g., benzalkonium chloride, benzethonium chloride, benzyl alcohol, cetylpyridinium chloride, chlorobutanol, phenol, phenylethyl alcohol, phenylmercuric nitrate, thimersol, and combinations thereof.

The pharmaceutical composition according to the present invention may also comprise an antioxidant, such as, e.g., ascorbyl pahnitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

The pharmaceutical composition according to the present invention may also comprise a surfactant, such as, e.g., polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc.

The pharmaceutical composition according to the present invention may also comprise acids or bases such as, e.g., hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof.

### Inhibition of the assembly of the HIV capsid

The compound of the present invention shall inhibit the assembly of the HIV capsid, in particular, of the HIV-1 capsid. Preferably, the term "capsid" refers to the protein shell of the HIV virus. The capsid of HIV is well known in the art, and, e.g. described by Engelman, A. and Cherepanov, P. Nature Reviews Microbiology 10, 279-290 (2012). A major component of the HIV capsid is the so called CA protein (capsid protein) which originated from the Gag polyprotein which is processed during maturation into structural proteins and viral enzymes.

Preferably, the pharmaceutical composition according to the present invention comprises at least one further antiretroviral agent as set forth herein below.

Moreover, the present invention relates to use of a compound of the present invention for the manufacture of a HIV capsid inhibitor, and, thus, for a medicament for inhibiting HIV capsid assembly.

Further envisaged by the present invention is method of treating a patient suffering from HIV comprising of administering a therapeutically effective amount of the compound or the pharmaceutical composition of the present invention to said subject.

The term "patient" as used herein, preferably, relates to a human. Moreover, the patient according to the present invention is preferably infected with the human immunodeficiency virus (HIV). As set forth elsewhere herein, the human immunodeficiency virus is a retrovirus that may cause acquired immunodeficiency syndrome (AIDS), a condition which may lead to life-threatening opportunistic infections. HIV in the context of the present invention, preferably, refers to HIV-1 and HIV-2. However, it is particularly contemplated that the term "HIV" refers to HIV-1.

Antiretroviral medicaments may be also used for pre-exposure and post-exposure prophylaxis (see Morbidity and Mortality Weekly Reports 2011;60 (03);65-68). Therefore, is it also contemplated that the compound or the pharmaceutical composition of the present invention is used for pre-exposure and post-exposure prophylaxis. Thus, the compound or the pharmaceutical composition of the present invention may also be administered to subjects which are not infected with HIV (in case of pre-exposure prophylaxis), or in subjects which are suspected of having contracted HIV recently (in case of post-exposure prophylaxis). Thereby, HIV infection may be prevented.

The compound or composition of the present invention may be administered by any way deemed appropriate. E.g., the compound or composition of the present invention is administered orally, parenterally, intracisternally, intrasternally, intravaginally, intraperitoneally or topically. Parental administration as used herein, preferably, refers to modes of administration which include intravenous, intramuscular, intraperitoneal, intracisternal, intarasternal, subcutaneous and intraarticular injection and infusion.

By way of example, the following preferred embodiments are mentioned:
1. A compound or a pharmaceutically acceptable salt or solvate thereof for use in inhibiting HIV capsid assembly, wherein the compound is a pyrimidine being at least substituted in two positions, preferably in 2 and 4 position or in 4 and 6 position, more preferably in 2 and 4 position.
2. The compound according to embodiment 1 having a structure according to formula (I) 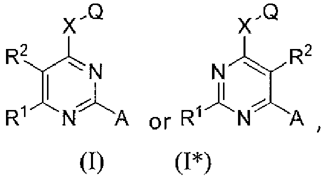 in particular according to formula (I) 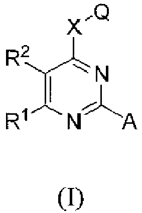 or a pharmaceutically acceptable salt or solvate thereof,
   wherein A is selected from the group consisting of aryl, heteroaryl and heterocycloalkyl group,
   wherein X is selected from the group consisting of O, N(R³) and S, and wherein wherein R³ is selected from the group consisting of H, alkyl, cycloalkyl, aryl, heteroaryl and heterocycloalkyl,
   wherein Q is selected from the group consisting of alkyl, cycloalkyl, aryl, heteroaryl and heterocycloalkyl,
   and wherein R¹ and R², are, independently of each other, selected from the group consisting of H, D, -alkyl, cycloalkyl, cycloalkenyl, heterocycloalkyl, aryl, heteroaryl, alkenyl, alkoxy and halides.
3. The compound according to embodiment 2, wherein A is a, substituted or unsubstituted, heteroaryl group, preferably a, substituted or unsubstituted, heteroaryl group comprising at least one N, more preferably a, substituted or unsubstituted, pyridine group.
4. The compound according to embodiment 2 or 3 , wherein A has the structure 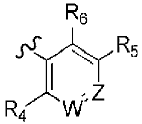 wherein W is N or C(R^{W}), and wherein Z is N or C(R^{Z}), and wherein R^{W} and R^{Z} are, independently of each other selected from the group consisting of H, alkyl, -O-alkyl, and halide,
   and wherein R⁴, R⁵ and R⁶, are, independently of each other, selected from the group consisting of -H, -alkyl, -O-alkyl, -halides.
5. The compound according to embodiment 4, wherein A has the structure 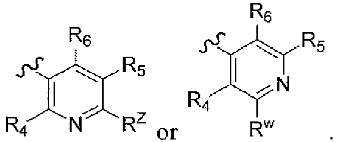
6. The compound according to embodiment 4 or 5, wherein R⁴, R⁵, R⁶, R^{W} and R^{Z} are, independently of each other selected from the group consisting of H, methyl, Br and Cl, preferably wherein A is selected from the group consisting of 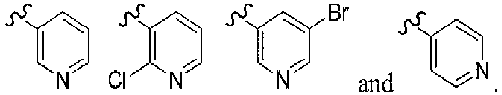
7. The compound according to embodiment 4 or 5, wherein X is NH or N(CH₃), more preferably NH.
8. The compound according to any one of embodiments 2 to 6, wherein Q is 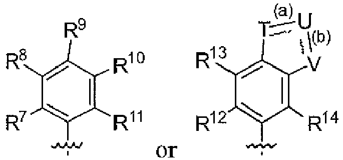 wherein R⁷, R⁸, R⁹, R¹⁰, R¹¹, R¹², R¹³ and R¹⁴ are, independently of each other, selected from the group consisting of -H, -alkyl, -halides, alkynyl, alkenyl, -NO₂ -CN, -C(=Y)-R¹⁵, -C(=Y)-H, -C(-Y)-Y¹-R¹⁵, -C(=Y)-OH,, -Y-R¹⁵, -NH₂, -NHR¹⁵,-NH -C(=Y)-R¹⁵,-CF₃, -S(O)R₁₅, -SO₂R¹⁵, P(O)R¹⁵R¹⁶, -P(O)(OR¹⁵)R¹⁶, -P(O)(OR¹⁵)(OR¹⁶), wherein Y is O, S or NH, and wherein Y¹ is O, S or NR*, wherein R* is H or alkyl, and wherein R¹⁵ and R¹⁶ are, independently of each other, selected from the group consisting of -H, - alkyl, -alkenyl, -heterocycloalkyl, aryl and heteroaryl,
   and wherein
   wherein T is -C-R^{T}, N or N-Y^{T}, with Y^{T} being H or alkyl
   wherein U is -C-R^{U}, N or N-Y^{U}, with Y^{U} being H or alkyl
   wherein V is -C-R^{V}, N or N-Y^{Y}, with Y^{V} being H or alkyl
   the bond (a) is a single or double bond,
   the bond (b) is a single or double bond,
   with eth proviso that one of (a) and (b) is a double bond,
   wherein R^{T}, R^{U}, and R^{V} are, independently of each other, H or -alkyl.
9. The compound according to embodiment 8, wherein Q is 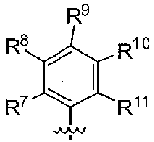 and wherein at least one of R⁷, R⁸, R⁹, R¹⁰ and R¹¹ is selected from the group consisting of -C(=O)-ethyl, -C(=O)-methyl -C(=O)-NH-CH₂-CH₂-F -C(=O)-H, -C(=O)-OH, -C(=O)-O-ethyl, -C(=O)-O-methyl -NH-C(=O)-OH, -O-methyl, -O-CH₂-CH₂-N(CH₂CH₃)₂, -NH-C(=Y)-(3-trifluoromethylphenyl)], -CF₃, -Cl and -F.
10. The compound according to embodiment 8 or 9, wherein Q is selected from the group consisting 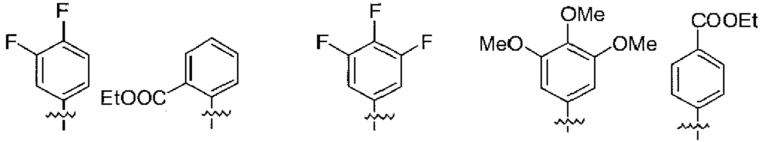 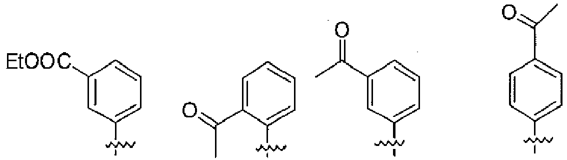 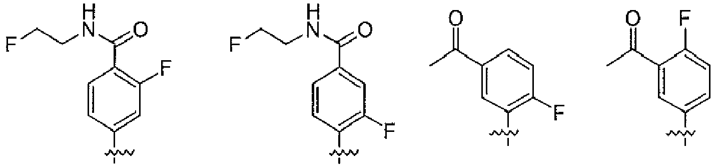 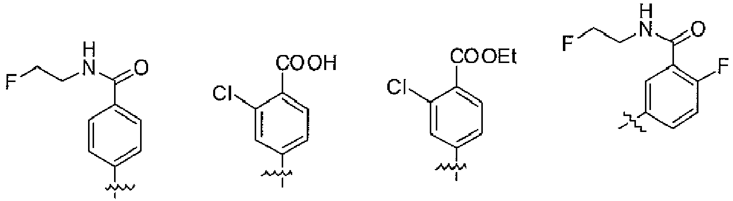 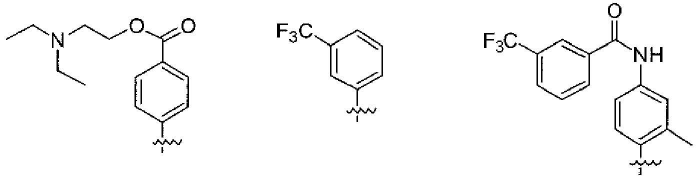
11. The compound according to embodiment 8, wherein Q is selected from the group consisting of 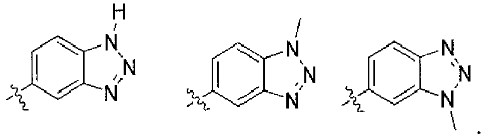
12. The compound according to embodiment 8, wherein Q is alkyl-F, preferably -CH₂-CH₂-F.
13. A compound according to any one of embodiments 1 to 12, wherein HIV is HIV-1 capsid assembly.
14. A pharmaceutical composition comprising a compound according to any one of embodiments 1 to 12.
15. Use of a compound as defined in any one of embodiments 1 to 12 for the manufacture of a medicament for inhibiting HIV capsid assembly.
16. A method of treating a patient suffering from HIV comprising administering a therapeutically effective amount of the compound as defined in any one of embodiments 1 to 12.

### Examples:

### I. General procedures of synthesis of the compounds according to the invention

### General procedure B

### 1.B : Nicotinimidamide hydrochloride

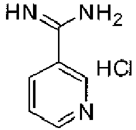

3-Cyanopyridine (20 g, 0.2 mol) was dissolved in MeOH (200 mL). Powdered NaOMe (1.1 g, 20 mmol) was added in one portion. The solution was stirred overnight at room temperature. After adding NH₄Cl (16.5 g, 0.31 mol), the mixture was heated at reflux for 4 h and then cooled. The solvent was removed in vacuo. Absolute EtOH (300 mL) was added and the mixture was heated to reflux. After 15 min, the solids were filtered off and the mixture was allowed to cool to room temperature and stand overnight. Additional inorganic salts were filtered off, and the reaction mixture was concentrated to approximately ½ volume and filtered to afford title compound (22.4, 74%).
¹H NMR (500 MHz, DMSO-d6) ä 9.5 (bs, 4H); 9.02 (dd, 1H, *J*= 2.5 Hz, *J*= 0.9 Hz); 8.86 (dd, 1H, *J* = 4.9 Hz, *J* = 1.6 Hz); 8.27 (ddd, 1H, *J* = 8.0 Hz, *J* = 2.5 Hz, *J* = 1.6 Hz); 7.64 (ddd, 1H, *J* = 8.0 Hz, *J* = 4.9 Hz, *J* = 0.9 Hz).
¹³C (125 MHz, DMSO-d6) δ 164.4; 154.2; 148.9; 136.5; 124.7; 123.9. HRMS: calcd for C₆H₇N₃, 121.0640; found, 121.0644.

### 2.B : 2-(pyridin-3-yl)pyrimidin-4(3H)-one

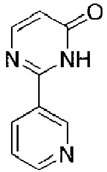

A solution of nicotinimidamide hydrochloride (3.8 g, 20.9 mmol) and sodium 3-ethoxy-3-oxoprop-1-en-1-olate (5.76 g, 41.8 mmol) in water (60 ml) was stirred at room temperature for 24 h. The reaction was quenched by the addition of 1M HCl until a pH = 7. The mixture was then extracted with dichloromethane (x3), dried over sodium sulfate, filtered, and concentrated in vacuo. A portion of the solid was subjected to recrystallization from ethyl-acetate/chloroform to give title compound (1.9 g 53 %) as white solid.
¹H NMR (400 MHz, DMSO-d6) δ 12.8 (vbs, 1H); 9.25 (bd, 1H, *J* = 2.2 Hz); 8.73 (dd, 1H, *J*= 4.8 Hz, *J* =1.6 Hz); 8.44 (ddd, 1H, *J* = 8.0 Hz, *J* = 2.3 Hz, *J* = 1.7 Hz); 8.16 (d, 1H, *J =* 8.0 Hz); 7.56 (ddd, 1H, *J*= 8.0 Hz, *J*= 4.8 Hz, *J*= 0.9 Hz); 6.43 (d, 1H, *J* = 6.5 Hz).
¹³C (125 MHz, DMSO-d6) δ 164.2; 157.5; 155.0; 152.1; 148.9; 135.5; 129.4; 123.8; 112.8. HRMS: calcd for C₉H₇N₃, 173.0590; found, 173.0590.

### 3.B : 4-chloro-2-(pyridm-3-yl)pyrimidme

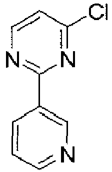

Applying heat to a mixture of 2-(pyridin-3-yl)pyrirnidin-4(3*H*)-one (0.53 g, 3.1 mmol) and POCl₃ (5.6 ml, 61.2 mmol) gave a solution which was refluxed for 1.5 h. Upon concentration of the reaction under vacuum, the oily residue was poured onto ice and stirred vigorously. After standing 1 h, the product was collected as a solid. Recrystallization from chloroform/hexane gave (0.33 g, 56%) as pale yellow solid
HRMS: calcd for C₉H₆N₃Cl, 191.0250; found, 191.0250.

### 2-(5-bromopyridin-3-yl)-4-chloropyrimidine (KP-346)

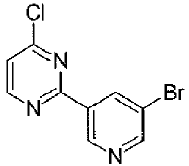

The title compound was prepared from 5-bromo-3-cyanopyridine (10 g, 54.6 mmol) according to general procedures B gave the title compound (8.8 g, 60% overall yield over tree steps).
¹H NMR (500 MHz, DMSO-d6) δ 9.41 (d, 1H, *J*= 1.8 Hz); 8.96 (d, 1H, *J* = 5.6 Hz); 8.92 (d, 1H, *J*= 2.3 Hz); 8.75 (dd, 1H, *J =* 2.3 Hz, *J=* 1.8 Hz); 7.78 (d, 1H, *J=* 5.3 Hz).
¹³C (125 MHz, DMSO-d6) δ 161.6; 161.0; 160.1; 152.9; 147.5; 137.6; 133.3; 121.5; 120.7. HRMS: calcd for C₉H₆N₃BrCl, 269.94281; found, 269.94257.

### General procedures A

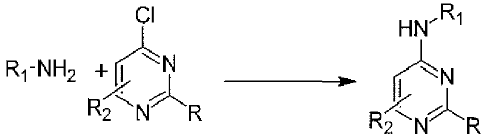

A mixture of starting pyrimidine derivative (1.0 mmol), aryl amine (1.2 mmol) in dioxane was stirred at 100 °C overnight. The mixture was allowed to cool to room temperature then was added a saturated solution of NaHCO₃ The crude reaction mixture was concentrated and purified using column chromatography (gradient: 50-80% ethyl acetate in hexane) to afford desired product as base, which was further converted to hydrochloric salt by treatment with HCl.

### Example 1: N-(3,4-difluorophenyl)-2-(pyridin-3-yl)pyrimidin-4-amine dihydrochloride (KP-343)

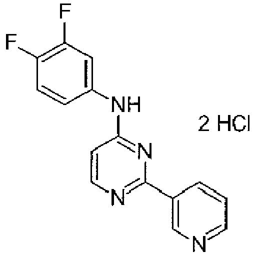

The title compound was prepared from 4-chloro-2-(pyridin-3-yl)pyrimidine (0.25 g, 1.30 mmol) and 3,4-difluoroaniline (0.155 ml, 1.57 mmol) by using an analogous procedure to that described in general procedure A and was isolated as yellow solid (0.302 g, 65 %).
¹H NMR (500 MHz, DMSO-d6) δ 11.52 (bs, 1H); 9.49 (d, 1H, *J* = 1.8 Hz); 9.14 (dm, 1H, *J* = 8.1 Hz); 9.05 (dd, 1H, *J* = 5.5 Hz, *J* = 1.1 Hz); 8.47 (d, 1H, *J* = 6.4 Hz); 8.14 (dd, 1H, J = 8.1 Hz, *J =* 5.5 Hz); 7.92 (ddd, 1H, *J* = 12.8 Hz, *J* = 7.3 Hz, *J* = 2.2 Hz); 7.59 (m, 1H); 7.44 (dt, 1H, *J =* 10.5 Hz, *J* = 9.1 Hz, *J* = 9.1 Hz); 7.17 (d, 1H, *J* = 6.4 Hz).
¹³C (125 MHz, DMSO-d6) δ 160.6; 157.3; 151.2; 149.2 (dd, *J* = 244.1 Hz, *J* = 13.4 Hz); 145.9 (dd, *J =* 242.5 Hz, *J* = 12.6 Hz); 145.6; 143.1; 142.6; 135.7 (dd, *J =* 9.0 Hz, *J* = 2.6 Hz); 133.5; 127.0; 117.8 (d, *J* = 17.9 Hz); 117.6; 110.1 (d, *J* = 21.3 Hz); 107.7.
HRMS: calcd for C₁₅H₁₁N₄F₂, 285.09463; found, 285.09413.

### Example 2: Ethyl 2-{[2-(pyridm-3-yl)pyrimidm-4-yl]amino}benzoate hydrochloride (KP-347)

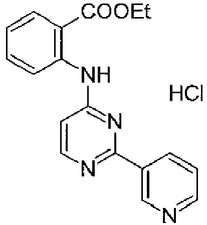

The title compound was prepared from 4-chloro-2-(pyridin-3-yl)pyrimidine (0.25 g, 1.30 mmol) and ethyl 2-aminobenzoate (0.231 ml, 1.57 mmol) by using an analogous procedure to that described in general procedure A and was isolated as white solid (0.326 g, 70 %).

¹H NMR (500 MHz, DMSO-d6) δ 10.65 (bs, 1H); 9.38 (d, 1H, *J* = 1.9 Hz); 8.91-8.94 (m, 2H); 8.51 (d, 1H, *J* = 6.1 Hz); 7.95 (dd, 1H, *J* = 8.0 Hz, *J* = 5.4 Hz); 7.91 (dd, 1H, *J* = 7.8 Hz, *J* = 1.6 Hz); 7.86 (dm, 1H, *J* = 8.2 Hz); 7.68 (m, 1 H); 7.32 (m, 1H); 7.01 (d, 1H, *J* = 6.1 Hz); 4.05 (q, 2H, *J*= 7.1 Hz); 1.07 (t, 3H, *J*= 7.1 Hz).
¹³C (125 MHz, DMSO-d6) δ 167.1; 160.6; 158.5; 154.4; 146.9; 144.5; 137.9; 133.9; 133.4; 130.7; 126.0; 124.5; 123.9; 123.3; 107.3; 61.1; 13.9.
HRMS: calcd for C₁₈H₁₇O₂N₄, 321.13460; found, 321.13465.

### Example 3: N-(3,4,5-trifluorophenyl)-2-(pyridin-3-yl)pyrimidin-4-amine dihydrochloride (KP-351)

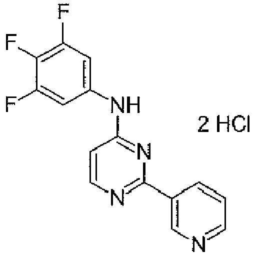

The title compound was prepared from 4-chloro-2-(pyridin-3-yl)pyrimidine (0.250 g, 1.30 mmol) and 3,4,5-trifluoroaniline (0.23 g, 1.57 mmol) by using an analogous procedure to that described in general procedure A and was isolated as light-yellow solid (0.352 g, 72 %).
¹H NMR (500 MHz, DMSO-d6) δ 10.95 (bs, 1H); 9.47 (bd, 1H, *J* = 1.9 Hz); 8.99 (dm, 1H, *J* = 8.0 Hz); 8.94 (dm, 1H, *J* = 5.3 Hz); 8.54 (d, 1H, *J* = 6.0 Hz); 7.99 (dd, 1H, *J* = 8.0 Hz, *J =* 5.3 Hz); 7.78 (m, 2H); 7.04 (d, 1H, *J*= 6.0 Hz).
¹³C (125 MHz, DMSO-d6) δ 160.3; 159-2; 155.3; 150.2 (ddd, *J*= 244.0 Hz, *J*= 10.0 Hz, *J* = 5.6 Hz); 146.7; 144.4; 140.4; 135.9 (m); 134.7; 134.5 (dt, *J* = 244.0 Hz, *J* = 15.5 Hz)_{;} 126.2; 107.8; 104.4.
HRMS: calcd for C₁₅H₁₀N₄F₃, 303.08521; found, 303.08518.

### Example 4: Ethyl 4-{[2-(5-bromopyridin-3-yl)pyrinudm-4-yl]amino}benzoate hydrochloride (KP-352)

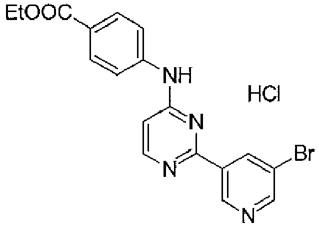

The title compound was prepared from 2-(5-bromopyridin-3-yl)-4-chloropyrimidine (0.25 g, 0.93 mmol) and ethyl 4-aminobenzoate (0.1884 g, 1.12 mmol) by using an analogous procedure to that described in general procedure A and was isolated as yellow solid (0.344 g, 85 %).
¹H NMR (500 MHz, DMSO-d6) δ 10.78 (bs, 1H); 9.41 (d, 1H, *J* = 1.8 Hz); 8.90 (d, 1H, *J* = 2.3 Hz); 8.78 (dd, 1H, *J =* 2.3 Hz, *J =* 1.8 Hz); 8.51 (d, 1H, *J =* 6.1 Hz); 8.00 (m, 2H); 7.92 (m, 2H); 7.03 (d, 1H, *J* = 6.1 Hz); 4.30 (q, 2H, *J* = 7.1 Hz); 1.33 (t, 3H, *J* = 7.1 Hz).
¹³C (125 MHz, DMSO-d6) δ 165.5; 160.4; 159.1; 153.9; 152.4; 147.5; 143.6; 137.9; 133.8; 130.5; 124.1; 120.6; 119.7; 107.7; 60.7; 14.4.
HRMS: calcd for C₁₈H₁₆O₂N₄ Br, 399.04511; found, 399.04523.

### Example 5: 2-(5-Bromopyridin-3-yl)-N-(3,4-difluorophenyl)pyrimidin-4-amine hydrochloride (KP-355)

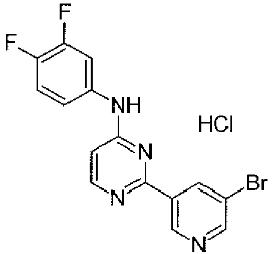

The title compound was prepared from 2-(5-bromopyridin-3-yl)-4-chloropyrimidine (0.25 g, 0.93 mmol) and 3,4-difluoroaniline (0.11 ml, 1.12 mmol) by using an analogous procedure to that described in general procedure A and was isolated as light-yellow solid (0.253 g, 68 %).
¹H NMR (500 MHz, DMSO-d6) δ 10.88 (bs, 1H); 9.36 (d, 1H, *J*= 1.9 Hz); 8.91 (d, 1H, *J*= 2.3 Hz); 8.76 (dd, 1H, *J =* 2.3 Hz, *J =* 1.9 Hz); 8.47 (d, 1H, *J* = 6.3 Hz); 7.98 (ddd, 1H, *J =* 13.3 Hz, *J* = 7.5 Hz, *J* = 2.5 Hz); 7.45-7-52 (m, 2H); 6.99 (d, 1H, *J* = 6.3 Hz).
¹³C (125 MHz, DMSO-d6) δ 160.4; 158.6; 152.6; 152.4; 149.2 (dd, *J*= 243.7 Hz, *J*= 13.1 Hz); 147.5; 145.8 (dd, *J* = 242.4 Hz, *J =* 12.9 Hz); 138.0; 135.9; 133.2; 120.5; 117.9 (d, *J*= 18.2 Hz); 117.4 (m); 110.0 (d, *J*= 21.5 Hz); 107.1.
HRMS: calcd for C₁₅H₁₀N₄ BrF₂, 363.00514; found, 363.00523.

### Example 6: 2-(5-Bromopyridin-3-yl)-N-(3,4,5-trifluorophenyl)pyrimidin-4-amine hydrochloride (KP-359)

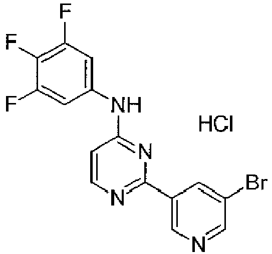

The title compound was prepared from 2-(5-bromopyridin-3-yl)-4-chloropyrimidine (0.25 g, 0.93 mmol) and 3,4,5-trifluoroaniline (0.164 g, 1.12 mmol) by using an analogous procedure to that described in general procedure A and was isolated as white solid (0.314 g, 80 %).
¹H NMR (500 MHz, DMSO-d6) δ 10.80 (bs, 1H); 9.34 (d, 1H, *J*= 1.9 Hz); 8.88 (d, 1H, *J* = 2.3 Hz); 8.71 (dd, 1H, *J =* 2.3 Hz, *J* = 1.9 Hz); 8.47 (d, 1H, *J* = 6.1 Hz); 7.71 (m, 2H); 6.95 (d, 1H, *J* = 6.1 Hz).
¹³C (125 MHz, DMSO-d6) δ 160.2; 159.1; 154.3; 152.3; 150.3 (ddd, *J=* 243.9 Hz, *J=* 10.1 Hz, J= 5.7 Hz); 147.3; 137.8; 133.6-135.8 (m, 2xC); 133.9; 120.6; 107.4; 104.6 (m).
HRMS: calcd for C₁₅H₈N₄BrF₃, 379-9884; found, 379.9879.

### Example 7: N-(3,4-difluorophenyl)-2-(pyridin-4-yl)pyrimidin-4-amine dihydrochloride (KP-360)

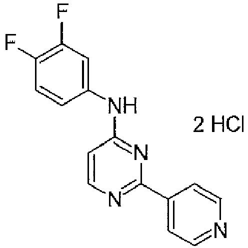

The title compound was prepared from 4-chloro-2-(pyridin-4-yl)pyrimidine (0.25 g, 1.30 mmol) and 3,4-difluoroaniline (0.155 ml, 1.56 mmol) by using an analogous procedure to that described in general procedure A and was isolated as light-yellow solid (0.308 g, 66 %).
¹H NMR (500 MHz, DMSO-d6) δ 10.68 (bs, 1H); 9.07 (m, 2H); 8.68 (m, 2H); 8.57 (d, 1H, *J*= 5.9 Hz); 7.95 (ddd, 1H, *J* = 13.1 Hz, *J*= 7.4 Hz, *J*= 2.6 Hz); 7.54 (m, 1H); 7.47 (dt, 1H, *J*= 10.5 Hz, *J* = 9.0 Hz); 7.06 (d, 1H, *J* = 6.0 Hz).
¹³C (125 MHz, DMSO-d6) δ 160.5; 158.6; 155.6; 152.0; 149.2 (dd, *J* = 243.9 Hz, *J*= 13.5 Hz); 143.6; 136.4 (dd, *J*= 9.1 Hz, *J*= 2.7 Hz); 124.6; 117.8 (d,*J* = 17.8 Hz); 116.9 (m); 109.5 (d, *J*= 21.4 Hz); 108.8.
HRMS: calcd for C₁₅H₁₀N₄F₂, 284-0874; found, 284.0877.

### Example 8: Ethyl 2-{[2-(5-bromopyridin-3-yl)pyrimidin-4-yl]amino}benzoate hydrochloride (KP-363)

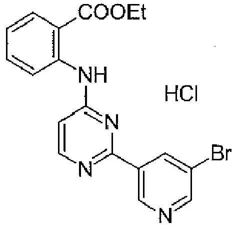

The title compound was prepared from 2-(5-bromopyridin-3-yl)-4-chloropyrimidine (0.25 g, 0.93 mmol) and ethyl 2-aminobenzoate (0.164 ml, 1.12 mmol) by using an analogous procedure to that described in general procedure A and was isolated as yellow solid (0.231 g, 57 %).
¹H NMR (500 MHz, DMSO-d6) δ 10.64 (bs, 1H); 9.29 (d, 1H, *J*= 1.9 Hz); 8.87 (d, 1H, *J* = 2.3 Hz); 8.68 (dd, 1H, *J* = 2.3 Hz, *J* = 1.9 Hz); 8.47 (d, 1H, *J* = 6.2 Hz); 7.91 (dd, 1H, *J*= 7.9 Hz, *J* = 1.6 Hz); 7.79 (dd, 1H, *J* = 8.2 Hz, *J* = 1.1 Hz); 7.68 (ddd, 1H, *J* = 8.2 Hz, J = 7.4 Hz, *J =* 1.6 Hz); 7.33 (m, 1H); 6.96 (d, 1H, *J =* 6.2 Hz); 4.05 (q, 2H*, J =* 7.1 Hz); 1.08 (t, 3H, *J* = 7.1 Hz).
¹³C (125 MHz, DMSO-d6) δ 166.9; 160.6; 158.6; 153.5; 152.4; 147.5; 137.8; 137.7; 133.3; 133.3; 130.6; 124.8; 124.2; 123.7; 120.5; 106.9; 61.1; 13.9.
HRMS: calcd for C₁₈H₁₆NO₂Br, 398.0378; found, 398.0378.

### Example 9: N-[2-(5-bromopyridin-3-yl)pyrimidin-4-yl]-1-methyl-1H-benzotriazol-5-amine dihydrochloride (KP-364)

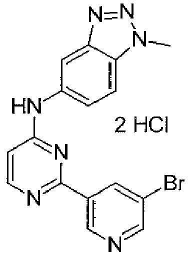

The title compound was prepared from 4-chloro-2-(pyridin-3-yl)pyrimidine (0.25 g, 0.93 mmol) and 1-methyl-1H-benzotriazol-5-amine (0.165 g, 1.12 mmol) by using an analogous procedure to that described in general procedure A and was isolated as light-yellow solid (0.309 g, 73 %).
¹H NMR (500 MHz, DMSO-d6) δ 11.04 (bs, 1H); 9.40 (d, 1H, *J* = 1.9 Hz); 8.94 (d, 1H, *J* = 2.3 Hz); 8.82 (dd, 1H, *J =* 2.3 Hz, *J =* 1.9 Hz); 8.52 (bs, 1H); 8.47 (d, 1H, *J =* 6.4 Hz); 7.91 (dd, 1H, *J* = 8.9 Hz, *J* = 0.7 Hz); 7.79 (dd, 1H, *J* = 8.9 Hz, *J* =1.8 Hz); 7.06 (d, 1H, *J* = 6.4 Hz); 4.32 (s, 3H).
¹³C (125 MHz, DMSO-d6) δ 161.8; 158.3; 152.8; 151.1; 147.6; 145.6; 138.0; 134.8; 130.8; 123.0; 120.5; 111.2; 109.7; 106.8; 34.5.
HRMS: calcd for C₁₆H₁₃N₇Br, 382.04103; found, 382.04118.

### Example 10: 4-Chloro-6-(2-chloro- pyridin-3-yl)pyrimidine

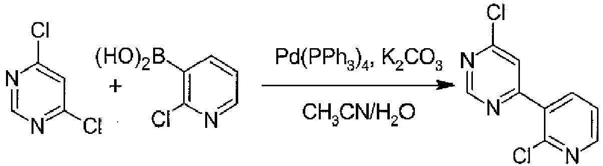

To 2-chloropyridine-3-boronic acid (1 g, 6.4 mmol) with 6-chloropyrimidine (1.9 g, 12.8 mmol), Pd(PPh₃)₄ (0.37, 0.32 mmol), and K₂CO₃ (1.8 g, 13.0 mmol) was added degassed MeCN and H₂O (1:1) as solvent (20 ml) and the reaction mixture was heated at 80°C for 2 hours in the capped flask under argon atmosphere. After cooling down the organic phase was separate and water phase was extracted with ethyl acetate (50 ml). The organic layers were combined and washed with saturated NaCl solution (50 ml). The organic layers were dried by MgSO₄ and evaporated under the vacuum. The resulting crude product was purified by silica gel chromatography with DCM/MeOH (97/3) to give 1.3 g (89 %) of title compound as white solid.

¹H NMR (300 MHz, DMSO-d6) *δ* 9.22 (s, 1H); 8.60 (dd, 1H, J= 2.1 Hz, *J =* 4.8 Hz); 8.17 (m, 1H); 8.15 (d, 1H, *J* = 1.8 Hz); 7.65 (m, 1H).

HRMS: calcd for C₉H₅N₃Cl, 224.98605; found, 224.98605

### Example 11 : Ethyl 4-{[6-(2-chloropyridin-3-yl)pyrimidin-4-yl]amino}benzoate hydrochloride (KP-369)

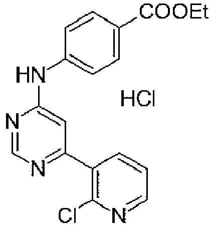

The title compound was prepared from 4-chloro-6-(2-chloropyridin-3-yl)pyrimidine (0.3 g, 1.32 mmol) and 2-aminobenzoate (0.263 g, 1.59 mmol) by using an analogous procedure to that described in general procedure A and was isolated as light-yellow solid (0.410 g, 79 %).
¹H NMR (500 MHz, DMSO-d6) δ 11.04 (bs, 1H); 8.95 (d, 1H, *J* = 1.1 Hz); 8.58 (dd, *J*= 4.8 Hz, *J* = 2.0 Hz); 8.15 (dd, *J* = 7.6 Hz, *J* = 2.0 Hz); 7.98 (m, 2H); 7.94 (m, 2H); 7.64 (dd, 1H, *J* =7.6 Hz, J= 4.8 Hz); 7.39 (d, 1H, *J* = 1.1 Hz); 7.30 (q, 2H, *J*= 7.1 Hz); 1.32 (t, 3H, *J* = 7.1 Hz).
¹³C (125 MHz, DMSO-d6) δ 165.5; 160.6; 157.2; 156.8; 151.1; 147.9; 143.5; 140.7; 131.6; 130.5; 124.4; 123.8; 119.8; 108.5; 60.7; 14.4.
HRMS: calcd for C₁₈H₁₆O₂N₄Cl, 355.09563; found, 355.09569.

### Example 12: 6-(2-Chloropyridin-3- yl)-N-(3,4,5-trifluorophenyl)pyrimidin-4-amine hydrochloride (KP-373)

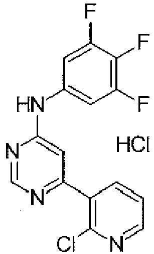

The title compound was prepared from 4-chloro-6-(2-chloropyridin-3-yl)pyrimidine (0.3 g, 1.32 mmol) and 3,4,5-trifluoroaniline (0.234 g, 1.59 mmol) by using an analogous procedure to that described in general procedure A and was isolated as light-yellow solid (0.322 g, 65 %).
¹H NMR (500 MHz, DMSO-d6) δ 11.35 (bs, 1H); 8.95 (d, 1H, *J =* 1.1 Hz); 8.58 (dd, 1H, *J*= 4.8 Hz, *J =* 1.9 Hz); 8.13 (dd, 1H, *J =* 7.6 Hz, *J =* 1.9 Hz); 7.78 (m, 2H); 7.64 (dd, 1H, *J =* 7.6 Hz, J = 4.8 Hz); 7.36 (d, 1H, *J* =1.1 Hz).
¹³C (125 MHz, DMSO-d6) δ 160.5; 156.9; 156.6; 151.2; 150.3 (ddd, *J* = 244.3 Hz, *J*= 9.9 Hz, *J* = 5.5 Hz); 147.9; 140.7; 135.5 (dt, *J* = 12.1 Hz, *J* = 3.1 Hz); 135.0 (dt, *J =* 245.2 Hz, *J =* 15.7 Hz); 131.3; 123.7; 108.3; 105.0.
HRMS: calcd for C₁₅H₉N₄ClF₃, 337.04624; found, 337.04628.

### Example 13: 6-(2-Chloropyridin-3-yl)-N-(3,4-difluorophenyl)pyrimidin-4-amine hydrochloride (KP-374)

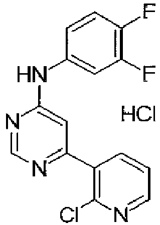

The title compound was prepared from 4-chloro-6-(2-chloropyridin-3-yl)pyrimidine (0.3 g, 1.32 mmol) and 3,4-difluoroaniline (0.16 ml, 1.59 mmol) by using an analogous procedure to that described in general procedure A and was isolated as white solid (0.377 g, 80 %).
¹H NMR (500 MHz, DMSO-d6) δ 11.51 (bs, 1H); 8.97 (d, 1H, *J*= 1.0); 8.61 (dd, 1H, *J =* 4.8 Hz, *J* = 1.9 Hz); 8-15 (dd, 1H, *J* = 7.6 Hz, *J =* 1.9 Hz); 8.01 (bddd, 1H, *J =* 12.9 Hz, *J* = 7.4 Hz, *J* = 2.3 Hz); 7.66 (dd, 1H, *J =* 7.6 Hz, *J* = 4.8 Hz); 7.45-7.53 (m, 2H); 7.36 (d, 1H, *J =* 1.0 Hz).
¹³C (125 MHz, DMSO-d6) δ160.8; 155.6; 154.6; 151.5; 149.1. (dd, *J* = 244.1 Hz, *J* = 13.3 Hz); 148.0; 146.2 (dd, *J* = 243.1 Hz, *J =* 12.6 Hz); 140.8; 135.4 (d, *J* = 7.6 Hz); 130.1; 123.7; 117.8; 117.9; 110.5 (d, *J* = 21.2 Hz); 108.0.
HRMS: calcd for C₁₅H₁₀N₄ClF₂, 319.05566; found, 319.05562.

### Example 14: N-[6-(2-Chloropyridin-3-yl)pyrimidin-4-yl]-1-methyl-1H-benzotriazol-5-amine hemihydrochloride (KP-375)

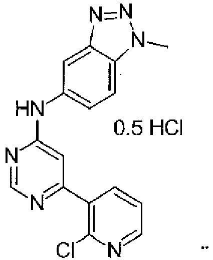

The title compound was prepared from 4-chloro-6-(2-chloropyridin-3-yl)pyrimidine (0.3 g, 1.32 mmol) and 1-methyl-1H-benzotriazol-5-amine (0.236 g, 1.59 mmol) by using an analogous procedure to that described in general procedure A and was isolated as yellow solid (0.326 g, 69 %).
¹H NMR (500 MHz, DMSO-d6) δ 10.93 (bs, 1H); 8.92 (d, 1H, *J*= 1.0 Hz); 8.59 (m, 1H); 8.58 (dd, 1H, *J*= 4.8 Hz, *J*= 1.9 Hz); 8.15 (dd, 1H, *J*= 7.6 Hz, *J=* 1.9 Hz); 7.87 (dd, 1H, *J*= 8.9 Hz, *J* = 0.6 Hz); 7.73 (dd, 1H, *J*= 8.9 Hz, *J* = 1.9 Hz); 7.64 (dd, 1H, *J* = 7.6 Hz, *J* = 4.8 Hz); 7.31 (d, 1H, *J*= 1.0 Hz); 4.30 (s, 3H).
¹³C (125 MHz, DMSO-d6) δ 160.9; 156.7; 151.1; 148.0; 145.7; 140.7; 135.1; 131.7; 123.7; 122.7; 111.2; 109.4; 107.7; 34.5.
HRMS: calcd for C₁₆H₁₂NClNa, 360.07349; found, 360.07348.

### Example 15: 6-(2-Chloropyridin-3-yl)-N-(3,4,5-trimethoxyphenyl)pyrimidin-4-amine hydrochloride (KP-377)

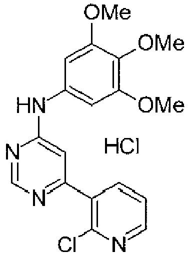

The title compound was prepared from 4-chloro-6-(2-chloropyridin-3-yl)pyrimidine (0.3 g, 1.32 mmol) and 3,4,5-trimethoxyaniline (0.292 g, 1.59 mmol) by using an analogous procedure to that described in general procedure A and was isolated as light-yellow solid (0.473 g, 87 %).
¹HNMR (500 MHz, DMSO-d6) δ 11.17 (bs, 1H); 8.93 (d, 1H, *J* = 1.0 Hz); 8.61 (dd, 1H, *J* = 4.8 Hz, *J* = 1.9 Hz); 8.14 (dd, 1H, *J* = 7.6 Hz, *J* = 1.9 Hz); 7.66 (dd, 1H, *J* = 7.6 Hz, *J* = 4.8 Hz); 7.28 (d, 1H, J= 1.0 Hz); 7.06 (bs, 2H); 3.79 (s, 6H); 3.66 (s, 3H).
¹³C (125 MHz, DMSO-d6) δ 161.0; 155.1; 153.5; 151.6; 148.0; 140.9; 135.0; 129.8; 123.7; 107.4; 100.0 60.4; 56.1.
HRMS: calcd for C₁₈H₁₇N₄ClNa, 395.08814; found, 395.08815.

### Example 16: N-(4-methyl-3-nitrophenyl)-3-(trifluoromethyl)-benzamide

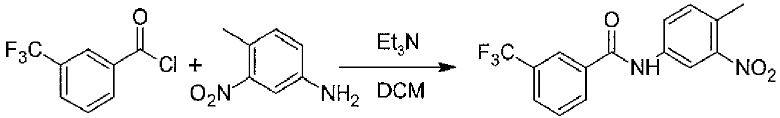

A solution of 4-methyl-3-nitro-phenylamine (3.64 g, 24 mmol) and 3-trifluoromethyl benzoyl chloride (5 g, 24 mmol) in DCM (100 ml) was treated with Et₃N (6.7 ml, 48 mmol). The mixture was stirred at 25 °C for 30 min. The reaction was then quenched with water (50 ml) and stirred for 15 min. The solid was collected by vacuum filtration and washed with hexane. A second crop of solid was collected from the filtrate to give a total yield of 7.78 g (96 %) of white-light yellow solid.

¹H NMR (500 MHz, DMSO-d₆) δ 10.75 (s, 1H); 8.51 (d, 1H, *J*= 1.6 Hz); 8.30 (s, 1H); 8.27 (d, 1H, *J*=7.8 Hz); 8.00 (m, 2H); 7.80 (t, 1H, *J* = 7.7 Hz); 7.48 (d, 1H, *J* = 8.4 Hz); 2.48 (s, 3H). ¹³C NMR (125 MHz,DMSO-d₆) 164.7; 148.9; 138.2; 135.6; 133.5; 132.4; 130.3; 129.9; 129.5; 129.0; 128.5; 125.4; 124.7; 116.1; 19.8.

HRMS: calcd for C₁₅H₁₁O₃N₂F₃Na, 347.0619, found 347.0621.

### Example 17: N-(3-Amino-4-methylphenyl)-3-(trifluoromethyl)benzamide

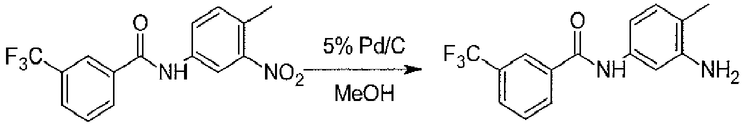

To a solution of *N*-(4-methyl-3-nitrophenyl)-3-(trifluoromethyl)-benzamide (3.0 g, 9.2 mmol) in methanol (40 mL) was added 5% Pd/C (500 mg). After two vacuum/H₂ cycles to replace air inside the reaction flask with hydrogen, the reaction mixture was stirred at room temperature under a hydrogen for 4 h. The reaction mixture was filtered through a pad of celite and concentrated under reduced pressure. The resulting crude product was purified by silica gel chromatography with hexane/ethyl acetate (1/5) to give 2.4 g (88 %) of title compound.

¹H NMR (500 MHz, DMSO-d₆) δ 10.17 (s, 1H); 8.27 (s, 1H); 8.25 (d, 1H, *J* = 7.9 Hz); 7.91 (d, 1H, *J* =7.8 Hz); 7.75 (t, 1H, *J*=7.7 Hz); 7.15 (s, 1H); 6.87 (m, 2H); 4.89 (s, 2H); 2.04 (s, 3H). ¹³C NMR(125 MHz, DMSO-d₆) δ 164.0; 147.1; 137.7; 136.6; 132.2; 130.2; 130.0; 129.8; 129-4; 128.2; 124.6; 117.7; 109.4; 107.0; 17.5.

HRMS: calcd for C₁₅H₁₃ON₂ F₃, 294.0980, found 294.0985.

### Example 18: N-(3-(6-(2-chloropyridin-3-yl)pyrimidin-4-ylamino)-4-methylphenyl)-3-(trifluoromethyl)benzamide hydrochloride (KP-380)

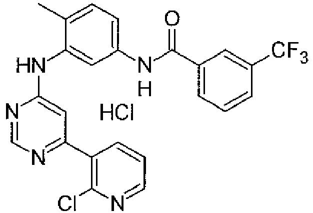

The title compound was prepared from 4-chloro-6-(2-chloropyridin-3-yl)pyrimidine (0.3 g, 1.32 mmol) and *N*-(3-Amino-4-methylphenyl)-3-(trifluoromethyl)benzamide (0.468 g, 1.59 mmol) by using an analogous procedure to that described in general procedure A and was isolated as yellow solid (0.504 g, 73 %).
¹H NMR (500 MHz, DMSO-d6) δ 11.08 (bs, 1H); 10.66 (s, 1H); 8.88 (bs, 1H); 8.61 (dd, 1H, *J* =4.8 Hz, *J* = 1.9 Hz); 9.31 (bs, 1H); 8.30 (dd, 1H, *J*= 4.8 Hz, *J* = 1.9 Hz); 8.31 (bs, 1H); 8.30 (m, 1H); 8.17 (dd, 1H, *J* = 7.7 Hz, *J* = 1.9 Hz); 7.96 (m, 1H); 7.95 (m, 1H); 7.78 (m, 1H); 7.65 (dd, 1H, *J* = 7.7 Hz, *J* = 4.8 Hz); 7.65 (m, 1H); 7.34 (d, 1H, *J* = 8.6 Hz); 7.17 (bs, 1H); 2.23 (s, 3H).
¹³C (125 MHz, DMSO-d6) δ 164.2; 162.4; 154.6; 153.2; 151.8; 148.0; 141.0; 137.6; 1.35.8; 135.2; 132.1; 131.2; 130.0; 129.5 (m, 2xC); 129.4 (q, *J* = 32.1 Hz); 128.4 (d, *J*= 2.6 Hz); 124.5 (d,*J* =3.4Hz); 124.2 (q, *J* = 272.4 Hz); 123.6; 119.6; 118.3; 10&.1; 17.6.
HRMS: calcd for C₂₄H₁₇ON₅ClF₃Na, 506.09659; found, 506.09651.

### Example 19: N-(3-(2-(5-bromopyridin-3-yl)pyrimidin-4-ylamino-4-methylphenyl)-3-(trifluoromethyl)benzamide hydrochloride (KP-384)

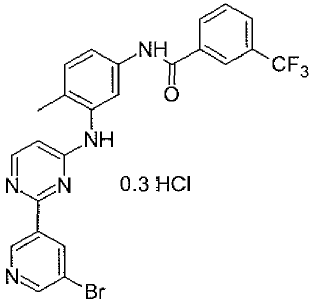

The title compound was prepared from 2-(5-bromopyridin-3-yl)-4-chloropyrimidme (0.3 g, 1.12 mmol) and *N*-(3-Amino-4-methylphenyl)-3-(trifluoromethyl)benzamide (0.394 g, 1.34 mmol) by using an analogous procedure to that described in general procedure A and was isolated as yellow foam (0.397 g, 66 %).
HRMS: calcd for C₂₄H₁₈ON₅BrF₃, 528.06413; found, 528.06418.

### Example 20: N-(3-(2-(pyridin-3-yl)pyritnidin-4-ylamino)-4-methylphenyl)-3-(trifluoromethyl)benzamide hydrochloride (KP-385)

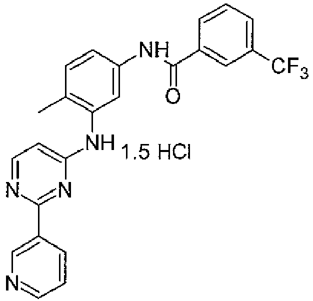

The title compound was prepared from 4-chloro-2-(pyridin-3-yl)pyrimidine (0.3 g, 1.57 mmol) and *N*-(3-amino-4-methylphenyl)-3-(trifluoromethyl)benzamide (0.553 g, 1.88 mmol) by using an analogous procedure to that described in general procedure A and was isolated as yellow solid (0.363 g, 65 %).
HRMS: calcd for C₂₄H₁₉ON₅F₃, 450.15362; found, 450.15341.

### General procedure C

A mixture of starting pyrimidine derivative (1.0 mmol), aryl amine (1.0 mmol) and HCl (1.0 mmol, solution in dioxane) in dioxane was stirred at 100 °C overnight. Then the mixture was allowed to cool to room temperature and a precipitate was collected by a suction filtration. The filtration cake was crystallized from methanol to give a desired product.

### Example 21: ethyl4-((2-(pyridin-3-yl)pyrimidin-4-yl)amino)benzoate hydrochloride (AMR-722)

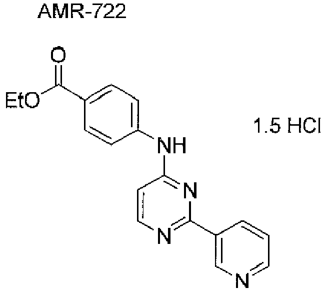

The title compound was prepared from 4-chloro-2-(pyridin-3-yl)pyrimidine (0.2 g, 1.047 mmol), ethyl 4-aminobenzoate (0.17 g, 1.047 mmol) and HCl (0.26 mL of 4 M solution in dioxane, 1.047 mmol) according to the general procedure C. A crystallization from methanol afforded 0.19 g (51 %) of solid; mp 153-154°C (methanol).
IR(KBr)v2531, 1701, 1642, 1579, 1512, 1414, 1368, 1278, 1174, 1109cm⁻¹
¹H NMR (400 MHz, CDCl₃) δ 10.85 (bs, 1H), 9.52 (s, 1H), 9.15 (d, *J* = 8.2 Hz, 1H), 8.98 (d, *J*= 4.8 Hz, 1H), 8.54 (d, *J* = 6.0 Hz, 1H), 8.06 (dd, *J* = 8.1, 5.5 Hz, 1H), 8.00 (d, *J* = 8.9 Hz, 2H), 7.96 (d, *J* = 8.9 Hz, 2H), 7.09 (d, *J*= 6.0 Hz, 1H), 4.29 (q, *J* = 7.1 Hz, 2H), 1.31 (t, *J* = 7.1 Hz, 3H).
¹³C NMR (100 MHz, CDCl₃) δ 165.7, 160.7, 158.2, 153.9, 145.1, 143.8, 142.9, 142.8, 135.0, 130.7 (2C), 127.4, 124.2, 119.8 (2C), 108.7, 60.9, 14.7.
HR-MS (TOF EI+) calcd. for C₁₈H₁₆N₄O₂ [M⁺] 320.1273, found 320.1266.

### Example 22: ethyl 3-((2-(pyridin-3-yl)pyridin-4-yl)amino)benzoate dihydrochloride

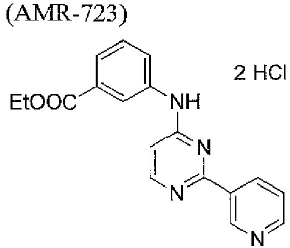

The title compound was prepared from 4-chloro-2-(pyridin-3-yl)pyrimidine (0.2 g, 1.047 mmol), ethyl 3-aminobenzoafie (0.17 g, 1.047 mmol) and HCl (0.26 mL of 4 M solution in dioxane, 1.047 mmol) according to the general procedure C. A crystallization from methanol afforded 0.20 g (53 %) of solid; mp181-183 °C (methanol).
IR (KBr) v 2550, 1723, 1632, 1499, 1397, 1377, 1280, 1212 cm⁻¹
¹H NMR (400 MHz, DMSO) δ 10.97 (s, 1H), 9.53 (d, *J* = 1.8 Hz, 1H), 9.11 (dt, *J* = 8.2, 1.7 Hz, 1H), 9.00 (dd, *J* = 5.4, 1.4 Hz, 1H), 8.62 (s, 1H), 8.52 (d, *J* = 6.2 Hz, 1H), 8.09-8.03 (m, 1H), 8.03-7.99 (m, 1H), 7.81-7.67 (m, 1H), 7.58 (t, *J =* 7.9 Hz, 1H), 7.08 (d, *J* = 6.3 Hz, 1H), 4.38 (q, *J* = 7.1 Hz, 5H), 1.34 (t, *J*= 7.1 Hz, 3H).
¹³C NMR (100 MHz, DMSO) δ 165.9, 160.9, 157.5, 151.1, 146.7, 143.9, 141.9, 139.3, 133.4, 130.9, 129.8, 126.8, 125.4, 124.7, 121.4, 108.1, 61.4, 14.7.
HR-MS (TOF EI+) calcd. for C₁₈H₁₆N4O₂ [M⁺] 320.1273, found 320.1278.

### Example 23: 1.-(4-((2-(pyridin-3-yl)pyrimidin-4-yl)amino)phenyl)ethanone hydrochloride (AMR-725)

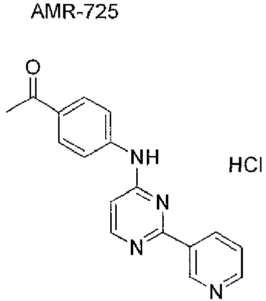

The title compound was prepared from 4-chloro-2-(pyridin-3-yl)pyrimidine (0.2 g, 1.047 mmol), 4-aminoacetophenone (0.14 g, 1.047 mmol) and HCl (0.26 mL of 4 M solution in dioxane, 1.047 mmol) according to the general procedure C. A crystallization from methanol afforded 0.14 g (37 %) of solid; mp155-158 °C (methanol).
IR (KBr) v 2633, 1678, 1644, 1602, 1506, I396, 1361, 1269, 1209, 1173 cm⁻¹.
¹H NMR (400 MHz, DMSO) δ 10.83 (s, 1H), 9.55 (d, *J*= 1.9 Hz, 1H), 9.25-9.10 (m, 1H), 9.01 (dd, *J* = 5.5, 1.3 Hz, 1H), 8.57 (d, *J*= 6.0 Hz, 1H), 8.10 (dd, *J* = 7.9, 5.5 Hz, 1H), 8.07-7.91 (m, 4H), 7.10 (d, *J*= 6.0 Hz, 1H), 2.57 (s, 3H).
¹³C NMR (100 MHz, CDCl₃) δ 196.8, 160.7, 158.1, 153.6, 145.2, 143.8, 143.0, 142.8, 134.9, 131.9, 129.9 (2C), 127.4, 119.8 (2C), 108.7,26.9.
HR-MS (TOF EI+) calcd. for C₁₇H₁₅ON₄ [M⁺] 290.1168, found 290.1175.

### Example 24: 1-(3-((2-(pyridin-3-yi)pyrimidin-4-yl)amino)phenyl)ethanone hydrochloride (AMR-726)

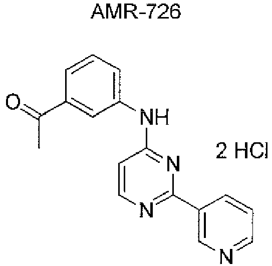

The title compound was prepared from 4-chloro-2-(pyridin-3-yl)pyrimidine (0.2 g, 1.047 mmol), 3-aminoacetophenone (0.14 g, 1.047 mmol) and HCl (0.26 mL of 4 M solution in dioxane, 1.047 mmol) according to the general procedure C. A crystallization from methanol afforded 0.12 g (31 %) of solid; mp 190-193 °C (methanol).
IR (KBr)v 2391, 1692, 1641, 1552, 1505, 1476, 1381, 1357, 1267, 1216 cm⁻¹.
¹H NMR (400 MHz, DMSO) δ 10.65 (s, 1H), 9.54 (d, *J* = 1.7 Hz, 1H), 9.22-9.07 (m, 1H), 8.97 (dd, *J* = 5.4, 1.5 Hz, 1H), 8.55 (s, 1H), 8.52 (d, *J*= 6.1 Hz, 1H), 8.02 (dd, *J*= 7.9, 5.4 Hz, 1H), 7.97 (dd, *J* = 8.1, 1.5 Hz, 1H), 7.83-7.68 (m, 1H), 7.59 *(t, J=* 7.9 Hz, 1H), 7.01 (d, *J*= 6.1 Hz, 1H), 2.64 (s, 3H).
¹³C NMR (100 MHz, DMSO) δ 198.0, 160.9, 157.6, 151.3, 146.4, 143.7, 142.2, 139.3, 137.8, 133.6, 129.8, 126.9, 125.5, 124.2, 120.3, 108.0, 27.3.
HR-MS (TOF EI+) calcd. for C₁₇H₁₅ON₄ [M⁺] 290.1168, found 290.1173.

### Example 25 : ethyl 4-((2-(pyridin-3-yl)-6-(trifluoromethyl)pyrimidin-4-yl)amino)benzoate hydrochloride (AMR-742)

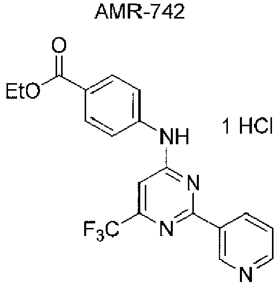

The title compound was prepared from 4-chloro-2-(pyridin-3-yl)-6-trifluoromethylpyrimidine (0.25 g, 0.96 mmol), ethyl 4-aminobenzoate (0.16 g, 0.96 mmol) and HCl (0.24 mL of 4 M solution in dioxane, 0.96 mmol) according to the general procedure C. A crystallization from methanol afforded 0.20 g (49 %) of solid; mp> 200 °C (methanol).
IR (KBr) ν 3065, 2996, 2418, 2101, 1696, 1637, 1599, 1514, 1426, 1369, 1277, 1181, 1150 cm⁻¹. ¹H NMR (400 MHz, DMSO) δ 11.02 (s, 1H), 9.04-8.83 (m, 2H), 8.58-8.31 (m, 2H), 8.04 (d, *J =* 8.8 Hz, 2H), 7.97 (d, *J* = 8.8 Hz, 2H), 7.40 (s, 1H), 4.30 (q, *J* = 7.1 Hz, 2H), 1.32 (t, *J* = 7.1 Hz, 3H).
¹³C NMR (100 MHz, DMSO) δ 165.7, 161.6, 160.6, 153.1 *(J*_{C-F}=35 Hz), 150.1, 144.9, 143.3, 130.7, 124.8, 124,7, 121.0 *(J*_{C-F} = 275 Hz), 119.9, 105.9, 60.9, 14.6.
HR-MS (ESI) calcd. for C₁₉H₁₆F₃O₂N₄ [M+H⁺] 389.1220, found 389.1220.

### Example 26: 1-(4-((2-(pyridin-3-yl)-6-(trifluoromethyl)pyrimidin-4-yl)amino)phenyl)ethanone hydrochloride (AMR-743)

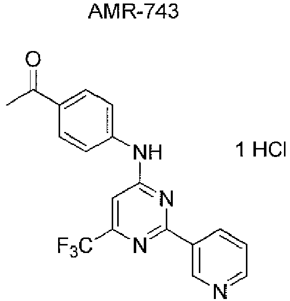

The title compound was prepared from 4-chloro-2-(pyridin-3-yl)-6-trifluoromethylpyrimidine (0.25 g, 0.96 mmol), 4-aminoacetophenone (0.13 g, 0.96 mmol) and HCl (0.24 mL of 4 M solution in dioxane, 0.96 mmol) according to the general procedure C. A crystallization from methanol afforded 0.23 g (60 %) of solid; mp> 200 °C (methanol.
IR (KBr) ν 2568, 1672, 1593, 1513, 1423, 1374, 1274, 1184, 1150, 995 cm⁻¹.
¹H NMR (400 MHz, DMSO) δ 11.26 (s, 1H), 9.01 (d, *J* = 6.6 Hz, 2H), 8.61 (d, *J* = 6.6 Hz, 2H), 8.03 (d, *J*= 8.8 Hz, 2H), 7.98 (d, *J*= 8.8 Hz, 2H), 7.50 (s, 1H), 2.56 (s, 3H).
¹³C NMR (100 MHz, DMSO) δ 196.5, 161.3, 160.5, 152.8 (*J*_{C-F}=25 Hz) 149.4, 145.0, 142.9, 131.8, 129.7, 124.2, 120*.*5(*J*_{C-F}= 274 Hz), 119.5, 105.5, 26.5.
HR-MS (ESI) calcd. for C₁₈H₁₄F₃ON₄ [M+H⁺] 359.1114, found 359.1114.

### General procedure D

A mixture of starting pyrimidine derivative (1.0 eq.), aryl amine (1.0 eq.) and HCl (1.0 eq., solution in dioxane) in dioxane was stirred at 100 °C overnight. The mixture was allowed to cool to room temperature then was diluted with water and aqueous ammonia and a saturated solution of NaHCO₃ was added stepwise. The product was extracted with dichloromethane and combined organic extracts were washed with water, brine and dried over MgSO₄, and concentrated *in vacuo.* A column chromatography of a residue afforded product as a base which was further converted to hydrochloride salt by treatment with HCl.

### Example 27: ethyl 4-((2-(pyridin-4-yl)pyrimidin-4-yl)amino)benzoate hydrochloride (AMR-747)

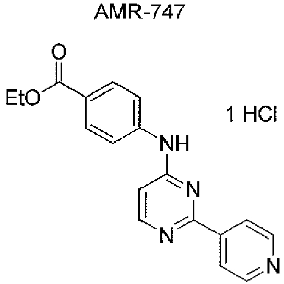

The title compound was prepared from 4-chloro-2-(pyridin-4-yl)-pyrimidine (0.41 g, 2.14 mmol), ethyl 4-aminobenzoate (0.35 g, 2.14 mmol) and HCl (0.53 mL of 4 M solution in dioxane, 2.14 mmol) according to the general procedure D. A column chromatography (eluent EtOAc:methanol/10:1) yielded 0.55 g (80%) of base which was converted to hydrochloride salt by treatment with HCl.
Salt: mp 208-210 °C (methanol); IR (KBr) v 3269, 3078, 2535, 1696, 1579, 1513, 1469, 1360, 1274, 1178, 1110, 988 cm⁻¹.
¹H NMR (400 MHz, DMSO) δ 10.67 (bs, 1H), 9.00 (d, *J* = 5.4 Hz, 2H), 8.68 (d, J= 5.4 Hz, 2H), 8.60 (dd, *J* = 5.9, 1.4 Hz, 1H), 8.01 (d*, J* = 8.9 Hz, 2H), 7.96 (d*, J* = 8.9 Hz, 2H), 7.09 (d*, J* = 5.9 Hz, 1H), 4.36-4.15 (m, 2H), 1.31 (td, *J* = 7.1, 1.4 Hz, 3H).
¹³C NMR (100 MHz, DMSO) δ 165.8, 160.6, 158.7, 156.1, 152.3, 144.2, 143.6 (2C), 130.7 (2C), 124.9 (2C), 123.8, 119.3 (2C), 109.7, 60.9, 14.7.
HR-MS (ESI) calcd. for C₁₈H₁₇O₂N₄ [M+H⁺] 321.1346, found 321.1346.

### Example 28: 1-(4-((2-(pyridin-4-yl)pyrimidin-4-yl)amino)phenyl)ethanone hydrochloride (AMR-748)

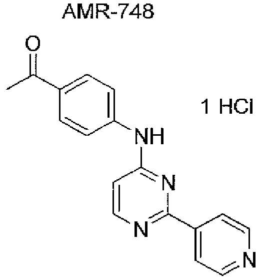

The title compound was prepared from 4-chloro-2-(pyridin-4-yl)-pyrimidine (0.30 g, 1.56 mmol), 4-aminoacetophenone (0.21 g, 1.56 mmol) and HCl (0.39 mL of 4 M solution in dioxane, 1.56 mmol) according to the general procedure D. A column chromatography (eluent EtOAc:methanol/10:1) yielded 0.14 g (31%) of base which was converted to hydrochloride salt by treatment with HCl. Salt: mp> 200 °C (methanol); IR (KBr) v 2640, 1677, 1639, 1604, 1546, 1485, 1398, 1356, 1275, 1208 cm⁻¹.
¹H NMR (400 MHz, DMSO) δ 10.74 (bs, 1H), 9.02 (d, *J* = 6.2 Hz, 2H), 8.72 (d, *J* = 6.2 Hz, 1H), 8.61 (d, *J* = 5.9 Hz, 1H), 8.02 (d, J= 8.8 Hz, 1H), 7.96 (d, J= 8.8 Hz, 2H), 7.12 (d, *J* = 5.9 Hz, 1H), 2.54 (s, 3H).
¹³C NMR (100 MHz, DMSO) δ 196.8, 160.6, 159.6, 156.5, 150.4, 145.7 (2C), 144.4, 131.4, 130.1 (2C), 124.1 (2C), 119.3, 109.3 (2C), 26.9.
HR-MS (ESI) calcd. for C₁₇H₁₅ON₄ [M+H⁺] 291.1240, found 291.1242.

### General procedure E

A mixture of starting pyrimidine derivative (1.0 eq.), amine (1.1 eq.) and NaHCO₃ (2.5 eq.) in DMF was stirred at 60 °C overnight. The mixture was allowed to cool to room temperature and then was quenched with water. The product was extracted with dichloromethane and combined organic extracts were washed with water, brine and dried over MgSO₄, and concentrated in *vacuo.* A column chromatography of a residue afforded product as a base which was further converted to hydrochloride salt by treatment with HCl.

### Example 29: N-(2-fluoroethyl)-2-(pyridin-4-yl)pyrimidin-4-amine hydrochloride (AMR-758)

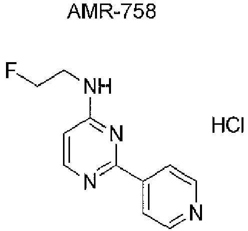

The title compound was prepared from 4-chloro-2-(pyridin-4-yl)-pyrimidine (0.43 g, 2.24 mmol), fluoroethylamine hydrochloride (0.25 g, 2.47 mmol) and NaHCO₃ (0.48 g, 5.61 mmol) according to the general procedure E. A column chromatography (eluent EtOAc) yielded 0.3 g (61 %) of base which was converted to hydrochloride salt by treatment with HCl.
Base; mp 145-148 °C (methanol); ¹H NMR (400 MHz, DMSO) δ 8.77-8.57 (m, 2H), 8.22 (d, *J* = 5.4 Hz, 1H), 8.16 (dd, *J* = 4.5, 1.5 Hz, 2H), 7.84 (s, 1H), 6.57 (d, *J* = 5.9 Hz, 1H), 4.61 (dt, *J* = 47.6, 5.0 Hz, 2H), 3.75 (m, 2H).
Salt; mp 178-181°C (methanol); IR (KBr) v 2548, 1643, 1549, 1417, 1357, 1200, 1038, 821 cm⁻¹.
¹H NMR (400 MHz, DMSO) δ 8.95 (d, *J* = 5.3 Hz, 2H), 8.78 (m, 1H), 8.58 (m, 2H), 8.31 (m, 1H), 6.80 (s, 1H), 4.82 -4.44 (m, 2H), 3.85 (m, 2H).
¹³C NMR (100 MHz, DMSO) δ 162.9, 157.8, 150.5, 148.1, 145.7 (2C), 124.11(2C),107.2, 82.4 (*J*_{C-F}= 165.5 Hz), 41.4, (*J*_{C-F}= 20.3 Hz); HR-MS (ESI) calcd. for C₁₁H₁₂FN₄ [M+H⁺] 219.1041, found 219.1040.

### Example 30: ethyl 3-((2-(pyridin-4-yl)pyrimidin-4-yl)amino)benzoate hydrochloride (AMR-759)

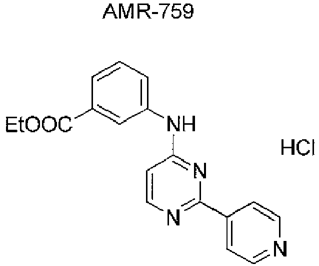

The title compound was prepared from 4-chloro-2-(pyridin-4-yl)-pyrimidine (0.20 g, 1.07 mmol), ethyl 3-aminobenzoate (0.17 g, 2.14 mmol) and HCl (0.27 mL of 4 M solution in dioxane, 1.07 mmol) according to the general procedure D. A column chromatography (eluent EtOAc:methanol/10:1) yielded 0.27 g (79%) of base which was converted to hydrochloride salt by treatment with HCl.
Salt: mp > 200 °C (methanol); IR (KBr) v 3407, 2568, 1715, 1635, 1579, 1479, 1439, 1362, 1282, 1273, 1212, 988 cm⁻¹.
¹H NMR (400 MHz, DMSO) δ 10.57 (bs, 1H), 9.01 (d, *J* = 6.4 Hz, 2H), 8.67 (d, *J* = 6.4 Hz, 2H), 8.59 (s, 1H), 8.56 (d, *J*= 5.9 Hz, 1H), 8.08-7.91 (m, 1H), 7.67 (d, *J*= 7.7 Hz, 1H), 7.54 (t, *J*= 7.9 Hz, 1H), 7.03 (d, *J*= 6.0 Hz, 1H), 4.36 (q, *J*= 7.1 Hz, 3H), 1.32 (t, *J*= 7.1 Hz, 3H). ¹³C NMR (100 MHz, DMSO) δ 166.0,160.7, 158.5, 155.2, 151.9, 143.6, 139.9, 130.8, 129.7, 124.6, 123.8, 120.6, 109.2, 61.3 14.7.
HR-MS (ESI) calcd. for C₁₈H₁₇N₄O₂ [M+H⁺] 321.1346, found 321.1347.

### Example 31: N-(2-fluoroethyl)-4-((2-(pyridin-4-yl)pyrimidin-4-yl)amino)benzamide hydrochloride (AMR-763)

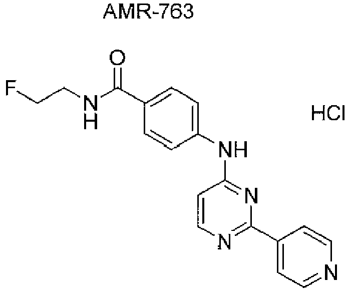

The title compound was prepared from 4-chloro-2-(pyridin-4-yl)-pyrimidine (0.26 g, 1.37 mmol), 2-fluoroethyl 4-aminobenzamide (0.25 g, 1.37 mmol) and HCl (0.34 mL of 4 M solution in dioxane, 1.36 mmol) according to the general procedure D. A column chromatography (eluent DCM:methanol/10:1) yielded 0.14 g (30%) of base which was converted to hydrochloride salt by treatment with HCl.
Base; mp > 200 °C (methanol); ¹H NMR (400 MHz, DMSO) δ 10.08 (s, 1H), 8.76 (d, *J* = 6.1 Hz, 2H), 8.59 (t, *J*= 5.6 Hz, 1H), 8.51 (d, *J* = 5.9 Hz, 1H), 8.31 - 8.13 (m, 2H), 7.93 (d, *J* = 8.9 Hz, 2H), 7.87 (d, *J*= 8.6 Hz, 2H), 6.89 (d, *J*= 5.9 Hz, 1H), 4.53 (dt, *J* = 47.2, 5.2 Hz, 2H), 3.56 (ddd, *J*= 26.8, 10.6, 5.3 Hz, 2H).
Salt; mp > 200 °C (methanol); IR (KBr) v 3034, 2516, 1652, 1606, 1505, 1490,1348,1305, 1210, 824 cm⁻¹.
¹H NMR (400 MHz, DMSO) δ 10.66 (s, 1H), 9.04 (d, *J*= 5.8 Hz, 2H), 8.72 (d, *J* = 5.6 Hz, 2H), 8.67 (t, *J*= 5.4 Hz, 1H), 8.59 (d, *J*= 5.9 Hz, 1H), 7.96 (d, *J* = 8.9 Hz, 2H), 7.89 (d, *J* = 8.5 Hz, 2H), 7.11 (d, *J*= 6.0 Hz, 1H), 4.54 (dt, *J* = 47.5, 5.1 Hz, 2H), 3.68-3.37 (m, 2H).
¹³C NMR (100 MHz, DMSO) δ 166.5, 160.8,158.3, 154.9, 152.1, 143.2, 142.2, 128.7, 125.1, 119.7, 109.4, 82.3 (*J*_{C-F}= 165.5 Hz), 40.4; ¹⁹F NMR (376.5 MHz, DMSO) δ -221.5.
HR-MS (ESI) calcd. for C₁₈H₁₇FON₅ [M+H⁺ 338.1412, found 338.1412.

### Example 32: 4-((2-(pyridin-3-yl)pyrimidin-4-yl)amino)-N-(2,2,2-trifluoroethyl)benzamide hydrochloride (AMR-771)

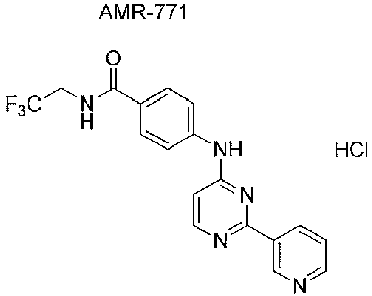

The title compound was prepared from 4-chloro-2-(pyridin-3-yl)-pyrimidine (0.26 g, 1.35 mmol), 2,2,2-trifluoroethyl 4-aminobenzamide (0.30 g, 1.35 mmol) and HCl (0.34 mL of 4 M solution in dioxane, 1.36 mmol) according to the general procedure D. A column chromatography (eluent DCM:methanol/10:1) yielded 0.27 g (54%) of base which was converted to hydrochloride salt by treatment with HCl.
Base; mp > 200 °C (methanol); ¹H NMR (400 MHz, DMSO) δ 10.06 (s, 1H), 9.48 (d, *J* = 1.3 Hz, 1H), 8.96 (t, *J* = 6.4 Hz, 1H), 8.70 (dd, *J*= 4.7, 1.6 Hz, 1H), 8.66-8.57 (m, 1H), 8.48 (d, *J* = 5.8 Hz, 1H), 7.95 (d, *J*= 9.0 Hz, 2H), 7.89 (d, *J*= 8.8 Hz, 2H), 7.55 (dd, *J* = 8.0, 4.8 Hz, 1H), 6.84 (d, *J*= 5.9 Hz, 1H), 4.19-3.95 (m, 2H).
Salt; mp> 200 °C (methanol); IR (KBr) v 2975, 1663, 1647, 1506, 1259, 1252, 1171, 1156, 835 cm⁻¹.
¹H NMR (400 MHz, DMSO) δ 10.78 (s, 1H), 9.52 (s, 1H), 9.08 (m, 2H), 8.97 (d, *J* = 4.3 Hz, 1H), 8.53 (d, *J* = 6.1 Hz, 1H), 8.08-8.01 (m, 1H), 7.99 (d, *J*= 8.8 Hz, 2H), 7.93 (d, *J =* 8.8 Hz, 2H), 7.08 (d, *J* = 6.1 Hz, 1H), 4.22-3.95 (m, 2H).
¹³C NMR (100 MHz, DMSO) δ 166.7, 160.8, 158.6, 154.0, 146.2, 143.8, 142.6, 141.9, 134.7, 129.1 (2C), 127.9, 126.9, 125.4 (*J*_{C*-*F} = 279.7 Hz), 119.9 (2C), 108.3, 40.4.
¹⁹F NMR (376.5 MHz, DMSO) δ -70.4.
HR-MS (ESI) calcd. for C₁₈H₁₅F₃ON₅ [M+H⁺] 374.1223, found 374.1223.

### Example 33: ethyl 4-((6-methyl-2-pyridin-3-yl)pyrimidin-4-yl)amino)benzoate hydrochloride (AMR-778)

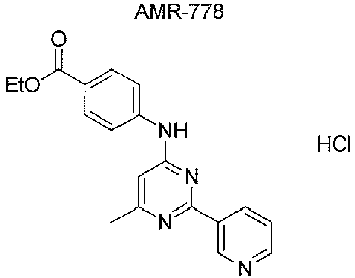

The title compound was prepared from 4-chloro-6-methyl-2-(pyridin-3-yl)-pyrimidine (0.39 g, 1.90 mmol), ethyl 4-aminobenzoate (0.31 g, 1.90 mmol) and HCl (0.47 mL of 4 M solution in dioxane, 1.90 mmol) according to the general procedure D. A column chromatography (eluent EtOAc:methanol/7:1) yielded 0.44 g (69%) of base which was converted to hydrochloride salt by treatment with HCl.
Base; mp 163-164 °C (methanol); ¹H NMR (400 MHz, DMSO) δ 10.09 (s, 1H), 9.55 (d, *J* = 2.0 Hz, 1H), 8.76 (d, *J*= 3.1 Hz, 1H), 8.70 (d, *J*= 7.9 Hz, 1H), 8.06 (d, *J*= 8.8 Hz, 2H), 7.99 (d, *J* = 8.8 Hz, 2H), 7.73-7.51 (m, 1H), 6.77 (s, 1H), 4.36 (q, *J*= 6.9 Hz, 2H), 2.56 (s, 3H), 1.40 (t, *J*= 6.9 Hz, 3H).
Salt; mp 196-199 °C (methanol); IR(KBr) v 2721, 1711, 1647, 1554, 1490, 1410, 1366, 1278, 1182, 1106, 1024 cm⁻¹.
¹H NMR (400 MHz, DMSO) δ 10.55 (s, 1H), 9.49 (d, *J* = 1.4 Hz, 1H), 9.04 (d, *J*= 8.1 Hz, 1H), 8.92 (d, *J* = 5.2 Hz, 1H), 7.99 (d, *J* = 8.9 Hz, 2H), 7.93 (d, *J*= 8.9 Hz, 2H), 7.92 (m, 1H), 6.86 (s, 1H), 4.29 (q, *J* = 7.1 Hz, 2H), 2.47 (s, 3H), 1.32 (t, *J* = 7.1 Hz, 3H).
¹³C NMR (100 MHz, DMSO) δ 165.8, 164.6, 160.8, 158.5, 145.8, 144.3, 143.6, 141.8, 135.0, 130.6 (2C), 126.8, 123.7, 119.4 (2C), 106.5, 60.8, 23.5, 14.7.
HR-MS (ESI) calcd. for C₁₉H₁₉O₂N₄ [M+H⁺] 335.1503, found 335.1503.

### Example 34: N-(2-fluoroethyl)-4-((2-(pyridin-3-yl)pyrimidin-4-yl)amino)benzamide hydrochloride (AMR-792)

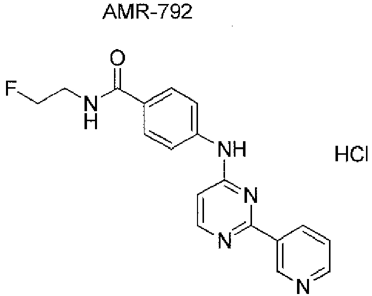

A mixture of 4-((2-(pyridin-3-yl)pyrmidin-4-yl)amino)benzoic acid (0.82 g, 2.4 mmol), 2-fluoroethylamine hydrochloride (0.33, 3.3 mmol), EDC.HCl (0.24, 1.23 mmol) and triethylamine (0.5 g, 4.9 mmol) in DMF was stirred at room temperature overnight. Then was the reaction quenched with water. The product was extracted with dichloromethane and combined organic extracts were washed with water, brine and dried over MgSO₄, and concentrated *in vacuo.* A column chromatography (eluent EtOAc:methanol/7:1) of a residue afforded 0.08 g (29%) of product as a base which was further converted to hydrochloride salt by treatment with HCl.
Salt; mp > 200 °C (methanol); IR (KBr) v 3011, 1651, 1535, 1504, 1360, 1306, 1211 cm⁻¹.
¹H NMR (400 MHz, DMSO) δ 11.13 (bs, 1H), 9.52 (d, *J* = 1.8 Hz, 1H), 9.22-9.11 (m, 1H), 9.02 (dd, *J* = 5.4, 1.3 Hz, 1H), 8.72 (t, *J* = 5.4 Hz, 1H), 8.52 (d, *J* = 6.2 Hz, 1H), 8.10 (dd, *J* = 8.1, 5.5 Hz, 1H), 7.96 (d, *J* = 8.8 Hz, 2H), 7.91 (d, J= 8.8 Hz, 2H), 7.16 (d, *J* = 6.2 Hz, 1H), 4.55 (dt, *J* = 47.5, 5.2 Hz, 2H), 3.57 (dq, *J* = 26.1, 5.2 Hz, 2H).
¹³C NMR (100 MHz, DMSO) δ 165.9, 160.4, 157.7, 152.6, 145.6, 143.2, 141.8, 141.4, 133.9, 128.8, 128.3, 126.6, 119.6, 107.7, 82.2 (*J*_{C-F} = 165.5 Hz), 39.8.
HR-MS (ESI) calcd. for C₁₈H₁₇OFN₅ [M+H⁺] 338.1412, found 338.1411.

### Example 35: 1-(4-((6-methyl-2-(pyridin-3-yl)pyrimidin-4-yl)amino)phenyl)ethanone hydrochloride (AMR-794)

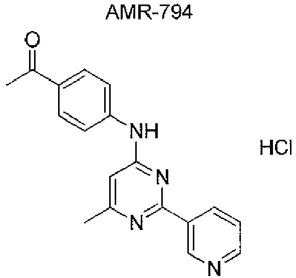

The title compound was prepared from 4-chloro-6-methyl-2-(pyridin-3-yl)-pyrimidine (0.33 g, 1.60 mmol), 4-aminoacetophenone (0.24 g, 1.77 mmol) and HCl (0.40 mL of 4 M solution in dioxane, 1.60 mmol) according to the general procedure D. A column chromatography (eluent EtOAc:methanol/7:1) yielded 0.16 g (33%) of base which was converted to hydrochloride salt by treatment with HCl.
Base; ¹H NMR (400 MHz, DMSO) δ 10.02 (bs, 1H), 9.55-9.36 (m, 1H), 8.69 (dd, *J*= 4.8, 1.6 Hz, 1H), 8.66-8.53 (m, 1H), 8.00 (d, *J* = 8.9 Hz, 2H), 7.92 (d, *J* = 8.9 Hz, 2H), 7.55 (dd, *J* = 8.0, 4.8 Hz, 1H), 6.70 (s, 1H), 2.54 (s, 3H), 2.43 (s, 3H).
Salt; mp 161-163 °C (methanol); ¹H NMR (400 MHz, DMSO) δ 10.87 (ds, 1H), 9.50 (d, *J* = 1.8 Hz, 1H), 9.16 (d, *J* = 8.1 Hz, 1H), 9.01 (dd, *J* = 5.4, 1.2 Hz, 1H), 8.10 (dd, *J* = 8.0, 5.5 Hz, 1H), 8.02-7.98 (m, 2H), 7.95 (d, *J*= 8.8 Hz, 2H), 6.96 (s, 1H), 2.55 (s, 3H), 2.49 (s, 3H).
¹³C NMR (100 MHz, DMSO) δ 196.3, 164.1, 160.5, 157.9, 144.9, 143.8, 142.9, 142.1, 134.7, 131.1, 129.6 (2C), 126.7, 119.2 (2C), 106.1, 26.4, 22.8.
HR-MS (ESI) calcd. for C₁₈H₁₇ON₄ [M+H⁺] 305.1397, found 305.1396.

### Example 36: N-(2-fluoroethyl)-4-((6-methyl-2-(pyridin-3-yl)pyrimidin-4-yl)amino)benzamide hydrochloride (AMR-795)

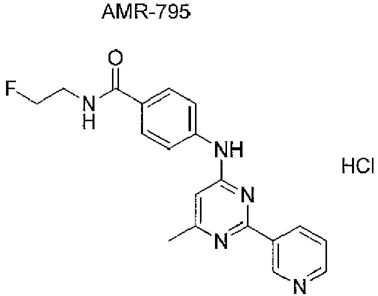

A mixture of 4-((6-methyl-2-(pyridin-3-yl)pyrimidin-4-yl)amino)benzoic acid (0.47 g, 1.53 mmol), 2-fluoroethylamine hydrochloride (0.76, 7.7 mmol), HBTU (0.88, 2.3 mmol) and triethylamine (1.54 g, 15.3 mmol) in DMF was stirred at room temperature overnight. Then was the reaction quenched with water. The product was extracted with dichloromethane and combined organic extracts were washed with water, brine and dried over MgSO₄, and concentrated *in vacuo.* A column chromatography (eluent EtOAc:methanol/7:1) of a residue afforded 0.21 g (39%) of product as a base which was further converted to hydrochloride salt by treatment with HCl.
Base; mp 203-204 °C (methanol) ¹H NMR (400 MHz, DMSO) δ 9.89 (s, 1H), 9.47 (d, *J*= 1.3 Hz, 1H), 8.68 (dd, *J*= 4.7, 1.7 Hz, 1H), 8.66-8.52 (m, 2H), 7.91 (d, *J* = 8.9 Hz, 2H), 7.89-7.76 (m, 2H), 7.63-7.50 (m, 1H), 6.67 (s, 1H), 4.53 (dt, *J*= 47.5, 5.1 Hz, 2H), 3.71 - 3.44 (m, 2H), 2.42 (s, 3H).
Salt; mp > 200 °C (methanol); IR (KBr) v 2585, 1650, 1583, 1504, 1458, 1368, 1257 cm-¹.
¹H NMR (400 MHz, DMSO) δ 10.83 (bs, 1H), 9.48 (d, *J*= 1.8 Hz, 1H), 9.11 (d, *J* = 8.1 Hz, 1H), 8.99 (dd, *J*= 5.4, 1.3 Hz, 1H), 8.69 (t, *J*= 5.5 Hz, 1H), 8.05 (dd, *J* = 8.0, 5.5 Hz, 1H), 7.94 (d, *J* = 8.8 Hz, 2H), 7.87 (d, *J*= 8.8 Hz, 2H), 6.93 (s, 1H), 4.55 (dt, *J* = 47.5, 5.3 Hz, 2H), 3.57 (dq, *J* = 26.1, 5.3 Hz, 2H), 2.49 (s, 3H).
¹³C NMR (100 MHz, DMSO) δ 165.9, 163.2, 160.6, 157.9, 145.7, 143.5, 141.7, 141.6, 134.1, 128.5, 128.3 (2C), 126.4, 119.5 (2C), 105.7, 82.2 (*J*_{C-F}= 165.6 Hz), 39.8, 22.45.
HR-MS (ESI) calcd. for C₁₉H₁₉OFN₅ [M+H⁺] 352.1568, found 352.1568.

### Example 37: ethyl 2-chloro-4-((2-(Pyrimidin-3-yl)pyrimidin-4-yl)amino)benzoate hydrochloride (AMR-808)

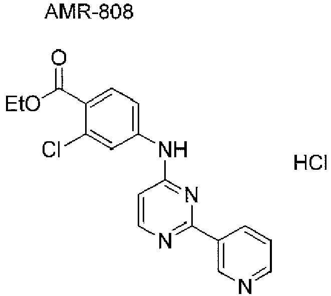

The title compound was prepared from 4-chloro-2-(pyridin-3-yl)-pyrimidine (0.30 g, 1.56 mmol), ethyl 4-amino-2-chlorobenzoate (0.31 g, 1.56 mmol) and HCl (0.39 mL of 4 M solution in dioxane, 1.60 mmol) according to the general procedure D. A column chromatography (eluent EtOAc:methanol/10:1) yielded 0.31 g (56%) of base which was converted to hydrochloride salt by treatment with HCl.
Base; ¹H NMR (400 MHz, DMSO) δ 10.24 (s, 1H), 9.47 (d, *J* = 1.3 Hz, 1H), 8.70 (dd, *J* = 4.8, 1.7 Hz, 1H), 8.61 (dd, *J*= 8.0, 1.9 Hz, 1H), 8.56-8.45 (m, 1H), 8.18 (d, *J* = 2.0 Hz, 1H), 7.93 (d, *J* = 8.7 Hz, 1H), 7.77 (dd, *J* = 8.7, 2.0 Hz, 1H), 7.65-7.49 (m, 1H), 6.85 (d, *J*= 5.8 Hz, 1H), 4.29 (q, *J* = 7.1 Hz, 2H), 1.31 (t, *J*= 7.1 Hz, 3H).
Salt; mp 199-201 °C (methanol); ¹H NMR (400 MHz, DMSO) δ 10.86 (s, 1H), 9.49 (d, *J*= 1.5 Hz, 1H), 9.04 (d, *J* = 8.1 Hz, 1H), 8.99-8.86 (m, 1H), 8.57 (d, *J* = 5.9 Hz, 1H), 8.07 (s, 1H), 8.01 (dd, *J*= 8.0, 5.4 Hz, 1H), 7.93 (d, *J* = 0.9 Hz, 2H), 7.06 (d, *J* = 6.0 Hz, 1H), 4.30 (q, *J* = 7.1 Hz, 2H), 1.32 (t, *J*= 7.1 Hz, 3H).
¹³C NMR (100 MHz, DMSO δ 64.6, 160.4, 159.3, 155.8, 146.6, 144.3, 144.1, 140.9, 135.1, 133.7, 132.9, 126.6, 122.7, 121.2, 117.9, 108.6, 61.3, 14.6.
HR-MS (ESI) calcd. for C₁₈H₁₆ClO₂N₄ [M+H⁺] 355.0956, found 355.0955.

### Example 38: 2-chloro-4-((2-(pyridin-3-yl)pyrimidin-4-yl)amino)benzoic acid hydrochloride (AMR-802)

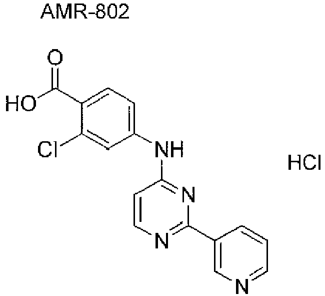

The title compound was prepared from 4-chloro-2-(pyridin-3-yl)-pyrimidine (0.40 g, 2.09 mmol), 4-amino-2-chlorobenzoic acid (0.39 g, 2.30 mmol) and HCl (0.52 mL of 4 M solution in dioxane, 2.20 mmol) according to the general procedure C. A crystallization from methanol yielded 0.62 g (91%) of acid; mp> 200 °C (methanol).
¹H NMR (400 MHz, DMSO) δ 10.74 (s, 1H), 9.50 (d, *J*= 1.2 Hz, 1H), 9.02 (d, *J* = 8.0 Hz, 1H), 8.97-8.85 (m, 1H), 8.57 (d, *J* = 6.0 Hz, 1H), 8.08 (d, *J* = 1.8 Hz, 1H), 8.02-7.96 (m, 1H), 7.94 (d, *J* = 8.7 Hz, 1H), 7.86 (dd, *J* = 8.7, 2.0 Hz, 1H), 7.03 (d, *J*= 6.0 Hz, 1H); ¹³C NMR (100 MHz, DMSO) δ
HR-MS (ESI) calcd. for C₁₆H₁₂ClO₂N₄ [M+H⁺] 327.0643, found 327.0643.

### General procedure F

A mixture of starting pyrimidine derivative (1.0 eq.), aminobenzoic acid (1.1 eq.) and two drops of HCl (conc.) in aqueous ethanol (4 mL ethanol with 2 mL of water) was stirred at reflux overnight. The mixture was then evaporated to dryness and was thoroughly dried *in vacuo* at 60 °C overnight. Next day an obtained solid dissolved in DMF (3 mL) and 2-fluoroethylamine hydrochloride (2.0 eq.), HBTU (1.0 eq) and triethylamine (8 eq.) were added stepwise.The reaction mixture was stirred at room temperature overnight. Then was the reaction quenched with water. The product was extracted with dichloromethane and combined organic extracts were washed with saturated solution of NaHCO₃, water, brine and dried over MgSO₄, and concentrated *in vacuo.* A column chromatography (eluent EtOAc:methanol/7:1) of a residue afforded 0.21 g (39%) of product as a base which was further converted to hydrochloride salt by treatment with HCl.

### Example 39: 2-fluoro-N-(2-fluoroethyl)-5-((2-(pyridin-3-yl)pyrimidin-4-yl)amino)benzamide dihydrochloride (AMR-816)

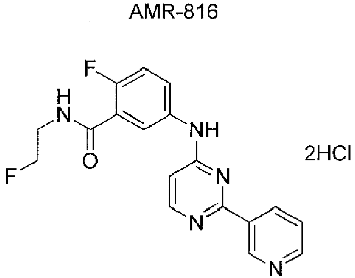

The title compound was prepared from 4-chloro-2-(pyridin-3-yl)-pyrimidine (0.30 g, 1.55 mmol), 2-fluoro-5-aminobenzoic acid (0.24 g, 1.55 mmol), HBTU (0.59 g, 1.55 mmol), fluoroethylamine hydrochloride (0.31 g, 3.1 mmol) and triethylamine (1.24 g, 12.3 mmol) according to the general procedure F. A column chromatography (eluent EtOAc:methanol/10:1) yielded 0.34 g (62%) of base which was converted to hydrochloride salt by treatment with HCl. Base; mp 170-174 °C (ethanol); ¹H NMR (400 MHz, DMSO) δ 9.91 (s, 1H), 9.47 (d, *J* = 1.5 Hz, 1H), 8.68 (dd, *J* = 4.7, 1.6 Hz, 1H), 8.62 (dd, *J* = 8.0, 1.9 Hz, 1H), 8.53 (m, 1H), 8.43 (d, *J* = 5.9 Hz, 1H), 8.20 (dd, *J* = 6.3, 2.8 Hz, 1H), 7.92-7.81 (m, 1H), 7.51 (dd, *J*= 7.9, 4.8 Hz, 1H), 7.41-7.23 (m, 1H), 6.75 (d, *J* = 5.9 Hz, 1H), 4.56 (dt, *J*= 47.4, 5.1 Hz, 2H), 3.59 (ddd, *J=* 26.1, 10.5, 5.2 Hz, 2H).
Salt; mp 179-182 °C (methanol); ¹H NMR (400 MHz, DMSO) δ 11.44 (bs, 1H), 9.53 (d, *J* = 1.7 Hz, 1H), 9.14 (dd, *J* = 6.7, 1.5 Hz, 1H), 9.02 (dd, *J* = 5.4, 1.1 Hz, 1H), 8.61 (d, *J* = 2.3 Hz, 1H), 8.49-8.40 (m, 1H), 8.19 (s, 1H), 8.05 (dd, *J* = 8.1, 5.4 Hz, 1H), 7.96-7.86 (m, 1H), 7.34 (t, *J* = 9.4 Hz, 1H), 7.16 (d, *J* = 6.5 Hz, 1H), 4.56 (dt, *J* = 47.4, 5.1 Hz, 2H), 3.61 (dq, *J* = 26.2, 5.2 Hz, 2H).
¹³C NMR (100 MHz, DMSO) δ 163.7, 160.4, 157.1, 155.31 (*J*_{C-F}= 246.7 Hz), 150.6, 146.2, 143.5, 141.7, 134.7, 132.9, 126.4, 124.6, 123.7 (*J*_{C-F} = 15.7 Hz), 122.1, 116.7 (*J*_{C-F} = 24.0 Hz), 107.2, 82.1 (*J*_{C-F} = 165.8 Hz), 40.1.
HR-MS (ESI) calcd. for C₁₈H₁₆FON₅ [M+H⁺] 356.1317, found 356.1317.

### Example 40: N-(2-(diethylamino)ethyl)-4-((2-(pyridin-3-yl)pyrimidin-4-yl)amino)benzamide dihydrochloride (AMR-822)

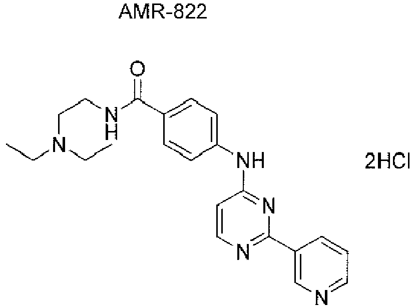

A mixture of 4-((2-(pyridin-3-yl)pyrimidin-4-yl)amino)benzoic acid (0.40 g, 1.37 mmol), N¹,N¹-diethylethane-1,2-diamine (0.32 g, 2.74 mmol), HBTU (0.57, 1.50 mmol) and triethylamine (0.69 g, 6.85 mmol) in DMF (3 mL) was stirred at room temperature overnight. Then was the reaction quenched with water. The product was extracted with dichloromethane and combined organic extracts were washed with water, brine and dried over MgSO₄, and concentrated *in vacuo.* A column chromatography (eluent DCM:methanol/5:1) of a residue afforded 0.43 g (80%) of product as a base which was further converted to hydrochloride salt by treatment with HCl.
Base; ¹H NMR (400 MHz, DMSO) δ 10.10 (s, 1H), 9.47 (s, 1H), 8.69 (d, *J* = 4.6 Hz, 1H), 8.61 (d, *J* = 8.0 Hz, 1H), 8.47 (d, *J* = 5.8 Hz, 1H), 8.33 (s, 1H), 7.88 (m, 4H), 7.55 (dd, *J* = 7.7, 4.9 Hz, 1H), 6.86 (d, *J*= 5.8 Hz, 1H), 3.34 (d, *J*= 6.2 Hz, 4H), 2.77 - 2.52 (m, 4H), 0.99 (t, *J* = 7.0 Hz, 6H).
Salt; mp 151-155 °C (methanol); ¹H NMR (400 MHz, DMSO) δ 11.04 (bs, 1H), 10.59 (bs, 1H), 9.50 (d, *J* = 1.5 Hz, 1H), 9.11 (d, *J* = 8.2 Hz, 1H), 8.98 (dd, *J* = 4.4, 3.1 Hz, 2H), 8.51 (d, *J* = 6.2 Hz, 1H), 8.10-8.03 (m, 1H), 8.01 (d, *J* = 8.8 Hz, 2H), 7.90 (d, *J*= 8.8 Hz, 2H), 7.14 (d, *J* = 6.2 Hz, 1H), 3.66 (m, 2H), 3.37 - 3.04 (m, 6H), 1.24 (t, *J* = 7.2 Hz, 6H).
¹³C NMR (100 MHz, DMSO) δ 166.0, 160.3, 158.1, 153.0, 146.0, 143.6, 141.8, 141.2, 133.9, 128.4 (2C), 128.1, 126.3,119.4 (2C), 107.7, 49.9, 46.7 (2C), 34.1, 8.4 (2C).
HR-MS (ESI) calcd. for C₂₂H₂₇ON₆ [M+H⁺] 391.2241, found 391.2241.

### General procedure G

A mixture of starting pyrimidine derivative (1.0 eq.), aryl amine (1.0 eq.) and HCl (conc., 3 drops) in aqueous ethanol (4 mL ethanol with 2 mL of water) was stirred reflux overnight. The mixture was allowed to cool to room temperature then was diluted with water and aqueous ammonia and a saturated solution of NaHCO₃ was added stepwise. The product was extracted with dichloromethane and combined organic extracts were washed with water, brine and dried over MgSO₄, and concentrated *in vacuo.* A column chromatography of a residue afforded product as a base which was further converted to hydrochloride salt by treatment with HCl.

### Example 41: ethyl 3-fluoro-4-((2-(pyridin-3-yl)pyrimidin-4-yl)amino)benzoate hydrochloride (AMR-823)

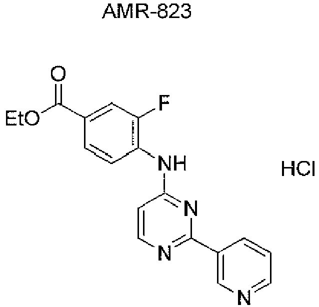

The title compound was prepared from 4-chloro-2-(pyridin-3-yl)-pyrimidine (0.36 g, 1.88 mmol), ethyl 4-amino-3-fluorobenzoate (0-38 g, 2.07 mmol) and HCl (cone., 3 drops) according to the general procedure G. A column chromatography (eluent DCM:methanol /10:1) yielded 0.20 g (31%) of base which was converted to hydrochloride salt by treatment with HCl.
Base; ¹H NMR (400 MHz, DMSO) δ 9.85 (s, 1H), 9.43 (d, *J*= 1.4 Hz, 1H), 8.69 (dd, *J* = 4.7, 1.6 Hz, 1H), 8.62 - 8.47 (m, 3H), 7.89 (d, *J* = 8.6 Hz, 1H), 7.78 (dd, *J*= 11.8, 1.7 Hz, 1H), 7.54 (dd, *J* = 7.4, 4.8 Hz, 1H), 7.07 (d, *J*= 5.8 Hz, 1H), 4.31 (q, *J=* 7.1 Hz, 2H), 1.33 (t, *J*= 7.1 Hz, 3H) Salt; mp 158-161 °C (methanol); ¹H NMR (400 MHz, DMSO) δ 10.39 (s, 1H), 9.45 (d, *J*= 1.9 Hz, 1H), 9.20-9.06 (m, 1H), 9.01 (dd,*J*=5.5, 1.2 Hz, 1H), 8.57 (d, *J*= 6.0 Hz, 1H), 8.39 (t, *J* = 8.3 Hz, 1H), 8.10 (dd, *J*= 8.1, 5.5 Hz, 1H), 7.87 (dd, *J*= 8.5, 1.9 Hz, 1H), 7.77 (dd, *J* = 11.5, 1.9 Hz, 1H), 7.23 (d, *J* = 6.0 Hz, 1H), 4.31 (q, *J* = 7.1 Hz, 2H), 1.33 (t, *J* = 7.1 Hz, 3H). ¹³C NMR (100 MHz, DMSO) δ 164.4, 160.4, 158.1, 155.2 (2C), 152.95 (*J*_{C-F}= 246.6 Hz), 144.6, 142.3 *(J*_{C*-*F} = 20.4 Hz), 134.9, 131.1 (*J*_{C-F} = 11.1 Hz), 126.9, 125.8 (*J*_{C-F} = 2.3 Hz), 125.5 (*J*_{C-F} = 6.6 Hz), 123.1, 116.1 (*J*_{C-F} = 21.2 Hz), 108.1, 108.1, 60.9, 14.1.
HR-MS (ESI) calcd. for C₁₈H₁₆FO₂N₄ [M+H⁺] 339.1252, found 339.1251.

### Example 42: 3-fluoro-N-(2-fluoroethyl)-4-((2-(pyridin-3-yl)pyrimidin-4-yl)amino)benzamide hydrochloride (AMR-825)

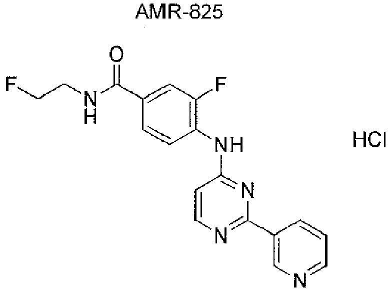

The title compound was prepared from 4-chloro-2-(pyridin-3-yl)-pyrimidine (0.30 g, 1.55 mmol), 3-fluoro-5-aminobenzoic acid (0.24 g, 1.55 mmol), HBTU (0.59 g, 1.55 mmol), fluoroethylamine hydrochloride (0.31 g, 3.1 mmol) and triethylamine (1.24 g, 12.3 mmol) according to the general procedure F. A column chromatography (eluent EtOAc:methanol/10:1) yielded 0.23 g (42%) of base which was converted to hydrochloride salt by treatment with HCl. Base; ¹H NMR (400 MHz, DMSO) 9.76 (s, 1H), 9.41 (d, *J=* 2.1 Hz, 1H), 8.73 (t, *J*= 5.6 Hz, 1H), 8.68 (dd, *J*= 4.8, 1.7 Hz, 1H), 8.59-8.53 (m, 1H), 8.50 (d, *J*= 5.8 Hz, 1H), 8.36 (t, *J*= 8.4 Hz, 1H), 7.79 (dd, *J*= 14.4,4.0 Hz, 2H), 7.53 (dd, *J =* 8.0, 4.8 Hz, 1H), 7.00 (d, *J =* 5.9 Hz, 1H), 4.54 (dt, *J*= 47.4, 5.1 Hz, 2H), 3.57 (ddd, *J* = 26.3, 10.5, 5.3 Hz, 2H).
Salt; mp 183-186 °C (methanol); ¹H NMR (400 MHz, DMSO) δ 10.16 (s, 1H), 9.43 (d, *J*= 1.4 Hz, 1H), 8.96 (d, *J*= 8.1 Hz, 1H), 8.93 - 8.88 (m, 1H), 8.82 (t, *J*= 5.4 Hz, 1H), 8.54 (d, *J =* 6.0 Hz, 1H), 8.24 (t, *J* = 8.4 Hz, 1H), 7.95 (dd, *J*= 8.0, 5.4 Hz, 1H), 7.85 (d, *J* = 4.3 Hz, 1H), 7.82 (s, 1H), 7.10 (d, *J =* 6.0 Hz, 1H), 4.55 (dt, *J*= 47.4, 5.1 Hz, 2H), 3.57 (ddd, *J*= 26.5, 10.5, 5.2 Hz, 2H).
¹³ C NMR (100 MHz, DMSO) δ 165.3, 161.0, 158.4, 154.7 (2C), 153.9 (*J*_{C-F} = 246.6 Hz), 145.6, 143.2, 142.2, 134.9, 131.2 (*J*_{C-F} = 6.2 Hz), 129.3 (*J*_{C-F} = 11.5 Hz), 127.1, 124.2 (*J*_{C-F} = 9.5 Hz), 115.1 (*J*_{C-F} = 21.2 Hz), 108.0, 82.5 (*J*_{C-F} = 165.8 Hz), 40.3.
HR-MS (ESI) calcd. for C₁₈H₁₆F₂ON₅[M+H⁺] 356.1317, found 356.1317.

### Example 43: 2-(diethylamino)ethyl 2-fluoro-4-{(2-(pyridin-3-yl)pyrimidin-4-yl)amino)benzoate hydrochloride (AMR-827)

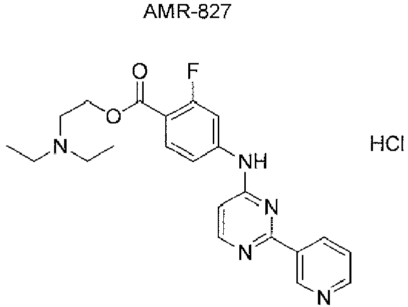

A mixture of 4-((2-(pyridin-3-yl)pyrimidin-4-yl)amino)benzoic acid (0.40 g, 1.37 mmol), 2-(diethylamino)ethanol (0.32 g, 2.74 mmol), HBTU (0.57, 1.50 mmol) and triethylamine (0.69 g, 6.85 mmol) in DMF (3 mL) was stirred at room temperature overnight. Then was the reaction quenched with water. The product was extracted with dichloromethane and combined organic extracts were washed with water, brine and dried over MgSO4, and concentrated *in vacuo.* A column chromatography (eluent DCM.-methanol/10:1) of a residue afforded 0.35 g (65%) of product as a base which was further converted to hydrochloride salt by treatment with HCl. Base; ¹H NMR (400 MHz, DMSO) δ 10.14 (s, 1H), 9.59-9.36 (m, 1H), 8.69 (dd, *J* = 4.5, 1.3 Hz, 1H), 8.67-8.57 (m, 1H), 8.49 (d, *J*= 5.8 Hz, 1H), 7.98 (d, *J*= 8.8 Hz, 2H), 7.92 (d, *J*= 8.8 Hz, 2H), 7.55 (dd, *J*= 8.0, 4.8 Hz, 1H), 6.85 (d, *J*= 5.9 Hz, 1H), 4.28 (t, *J*= 6.0 Hz, 2H), 2.76 (t, *J*= 5.9 Hz, 2H), 2.64 -2.35 (q, *J*= 7.1 Hz, 4H), 0.96 (t, *J* = 7.1 Hz, 6H).
Salt; mp 153-155 °C (methanol); ¹H NMR (400 MHz, DMSO) δ 11.06 (bs, 1H), 10.84 (bs, 1H), 9.51 (d, *J* = 1.5 Hz, 1H), 9.17-9. 05 (m, 1H), 8.98 (d, *J* = 4.3 Hz, 1H), 8. 54 (d, *J* = 6. 0 Hz, 1H), 8.14-7.91 (m, 4H), 7.15 (d, *J*= 6.1 Hz, 1H), 4.69-4-54 (m, 2H), 3.51 (d, *J =* 4.9 Hz, 2H), 3.34 - 3.06 (m, 4H), 1.27 (t, *J* = 7.2 Hz, 6H).
¹³B NMR (100 MHz, DMSO) δ 165.4, 160.7, 158.2, 154.1, 145.0, 144.3, 143.0, 142.7, 135.0, 131.1, 127-4, 123.4, 119.8, 108.7, 59.5, 49.7, 47.4 (2C), 8.9 (2C).
HR-MS (ESI) calcd. for C₂₂H₂₆O₂N₅ [M+H⁺] 392.2081, found 392.2079.

### Example 44: 2-fluoro-N-(2-fluoroethyl)-4-((6-methyl-2-(pyridin-3-yl)pyrimidin-4-yl)amino)benzamide hydrochloride (AMR-828)

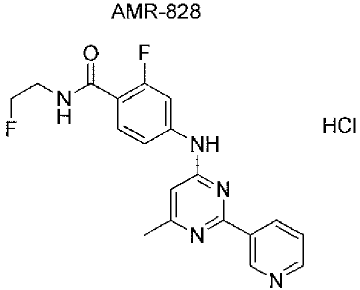

The title compound was prepared from 4-chloro-6-methyl-2-(pyridin-3-y1)-pyrimidine (0.41 g, 2.0 mmol), 4-amino-2-fluorobenzoic acid (0.31 g, 2.0 mmol), HBTU (0.76 g, 2.0 mmol), fluoroethylamine hydrochloride (0.40 g, 4.0 mmol) and triethylamine (1.62 g, 16.0 mmol) according to the general procedure F. A column chromatography (eluent EtOAc:methanol/7:1) yielded 0.16 g (22%) of base which was converted to hydrochloride salt by treatment with HCl. Base; ¹H NMR (400 MHz, DMSO) δ 10.06 (s, 1H), 9.46 (d, *J*= 1.4 Hz, 1H), 8.69 (dd, *J =* 4.8,
1.7 Hz, 1H), 8.59 (dt, *J* = 8.0, 1.9 Hz, 1H), 8.33 - 8.16 (m, 1H), 7.92 (dd, *J* = 14.0,1.9 Hz, 1H), 7.71 (t, *J* = 8.6 Hz, 1H), 7.55 (ddd, *J* = 8.0, 4.8, 0.7 Hz, 1H), 7.49 (dd, *J* = 8.6, 2.0 Hz,1H), 6.68 (s, 1H), 4.53 (dt, *J*= 47.4, 5.2 Hz, 2H), 3.56 (ddd, *J =* 26.0, 10.6, 5.3 Hz, 2H), 2.43 (s, 3H).
Salt; mp 184-187 °C (methanol); ¹H NMR (400 MHz, DMSO) δ 10.56 (bs, 1H), 9.47 (d, *J* = 1.6 Hz, 1H), 9.08-8.96 (m, 1H), 8.92 (dd, *J* = 5.3,1.4 Hz, 1H), 8.30 (dd, *J* = 9.0, 49 Hz, 1H), 7.97 (dd, *J* = 8.1, 5.4 Hz, 1H), 7.81 (dd, *J* = 13 .7, 1.8 Hz, 1H), 7.71 (t, *J* = 8.5 Hz, 1H), 7.60 (dd, *J* = 8.6, 2.0 Hz, 1H), 6.84 (s, 1H), 4.52 (dt, *J* = 47.5, 5.2 Hz, 2H), 3.56 (ddd, *J*= 26.3, 10.5, 5.1 Hz, 2H), 2.47 (s, 3H).
^{l3}c NMR (100 MHz, DMSO) δ δ 164.1, 163.5, 160.5, 159.6 (*J*_{C-F} = 247.6 Hz), 157.8, 144.6 (2C), 143.1 (*J*_{C-F} = 11.5 Hz), 142.3, 134.8, 131.0 (*J*_{C-F} = 3.6 Hz), 126.8, 116.8 (*J*_{C-F} = 13.7 Hz), 115.4, 106.6 (*J*_{C-F} = 28.0 Hz), 106.2, 82.1 (*J*_{C-F} = 165.7 Hz), 39.8, 22.8.
HR-MS (ESI) calcd. for C₁₉H₁₈F₂ON₅ [M+H⁺] 370.1474, found 370.1472.

### Example 45: 3-fluoro-N-(2-fluoroethyl)-4-((6-methyl-2-(pyridin-3-yl)pyrimidin-4-yl)amino)benzamide hydrochloride (AMR-829)

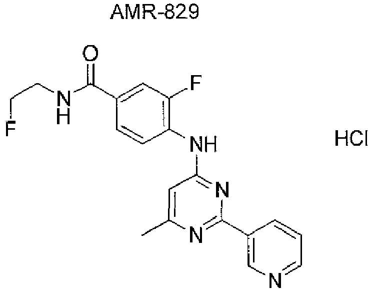

The title compound was prepared from 4-chloro-6-methyl-2-(pyridin-3-yl)-pyrimidme (0.41 g, 2.0 mmol), 4-amino-3-fluorobenzoic acid (0.31 g, 2.0 mmol), HBTU (0.76 g, 2.0 mmol), fluoroethylamine hydrochloride (0.40 g, 4.0 mmol) and triethylamine (1.62 g, 16.0 mmol) according to the general procedure F. A column chromatography (eluent EtOAc:methanol/7:1) yielded 0.16 g (22%) of base which was converted to hydrochloride salt by treatment with HCl. Base; ¹H NMR (400 MHz, DMSO) δ 9.62 (s, 1H), 9.41 (d, *J =* 2.1 Hz, 1H), 8.72 (t, *J*= 5.5 Hz, 1H), 8.67 (dd, *J* = 4.8, 1.7 Hz, 1H), 8.56 (dt, *J* = 8.0, 1.9 Hz, 1H), 8.33 (t, *J* = 8.5 Hz, 1H), 7-89 - 7.70 (m, 2H), 7.52 (dd, *J* = 8.0, 4.8 Hz, 1H), 6.84 (s, 1H), 4.54 (dt, *J* = 47.5, 5.1 Hz, 2H), 3.57 (ddd, *J* = 26.6, 10.4, 5.1 Hz, 2H), 2.42 (s, 3H).
Salt; mp 197-199 °C (methanol); ¹H NMR (400 MHz, DMSO) δ 10.10 (bs, 1H), 9.41 (d, *J*= 1.6 Hz, 1H), 8.99 (d, *J*= 8.2 Hz, 1H), 8.93 (dd, *J*= 5.4, 1.3 Hz, 1H), 8.81 (t, *J =* 5.5 Hz, 1H), 8.16 (t, *J*= 8.4 Hz, 1H), 7.98 (dd, *J*= 8.1, 5.5 Hz, 1H), 7.91 - 7.64 (m, 2H), 6.93 (s, 1H), 4.54 (dt, *J* = 47.4, 5.1 Hz, 2H), 3.57 (ddd, *J* = 26.4, 10.4, 5.2 Hz, 2H), 2.47 (s, 3H).
¹⁹F NMR (376.5 MHz, DMSO) δ -221.6, -123.1.
¹³C NMR (100 MHz, DMSO) δ 164.9, 164.1, 160.9, 157.6, 153.7 (*J*_{C-F} = 246.3 Hz), 144.8, 142.5, 142.2, 134.4, 130.7 (*J*_{C-F} = 5.3 Hz), 128.9 (*J*_{C-F} = 11.5 Hz), 126.7, 123.8, 114.7 (*J*_{C}-_{F} = 21.3 Hz), 105.6, 82.1 (*J*_{C}-_{F} = 165.8 Hz), 39.9, 22.6.
HR-MS (ESI) calcd. for C₁₉H₁₈F₂ON₅ [M+H⁺] 370.1474, found 370.1472.

### Example 46: 1-(3-((6-methyl-2-(pyridin-3-yl)pyrimidin-4-yl)amino)phenyl)ethanone hydrochloride (AMR-830)

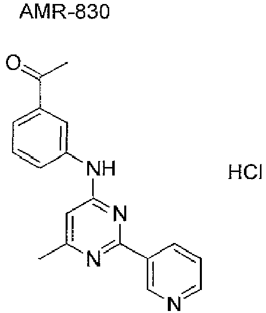

The title compound was prepared from 4-chloro-6-methyl-2-(pyridin-3-yl)-pyrimidine (0.33 g, 1.60 mmol), 3-aminoacetophenone (0.24 g, 1.77 mmol) and HCl (conc., 3 drops) according to the general procedure G A column chromatography (eluent DCM:methanol /10:1) yielded 0.41 g (84%) of base which was converted to hydrochloride salt by treatment with HCl.
Base; 177-178 °C (methanol); ¹H NMR (400 MHz, DMSO) δ 9.84 (s, 1H), 9.54-9.38 (m, 1H), 8.66 (m, 2H), 8.55 (s, 1H), 7.98-7.84 (m, 1H), 7.64 (d, *J*= 7.6 Hz, 1H), 7.59-7.41 (m, 2H), 6.62 (s, 1H), 2.60 (s, 3H), 2.41 (s, 3H).
Salt; mp> 200 °C (methanol); ¹H NMR (400 MHz, DMSO) δ 10.57 (bs, 1H), 9.47 (d, *J*= 1.5 Hz, 1H), 9.09-8.94 (m, 1H), 8.90 (dd, *J* = 5.2,1.4 Hz, 1H), 8.51 (s, 1H), 8.06-7.79 (m, 2H), 7.69 (d, *J* = 8.1 Hz, 1H), 7.53 (t, *J = 7.9* Hz, 1H), 6.81 (s, 1H), 2.60 (s, 3H), 2.46 (s, 3H).
¹³C NMR (100 MHz, DMSO) δ 198.0, 163.5, 161.0, 158.7, 147.5, 145.1, 140.2, 140.1, 137.7, 134.0, 129.6, 126.0, 124.9, 123.3, 119.7, 105.7, 27.2, 23.0.
HR-MS (ESI) calcd. for C₁₈H₁₇ON₄ [M+H⁺] 305.1379, found 305.1375.

### Example 47: 2-fluoro-N-(2-fluoroethyl)-4-((2-(pyridin-3-yl)pyrimidin-4-yl)amino)benzamide hydrochloride (AMR-831)

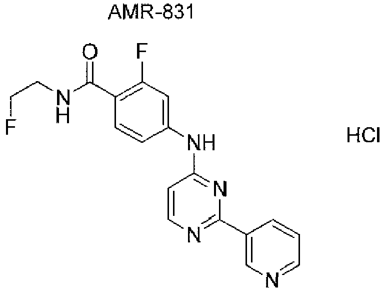

The title compound was prepared from 4-chloro-2-(pyridin-3-yl)-pyrimidine (0.38 g, 2.0 mmol), 4-amino-2-fluorobenzoic acid (0.31 g, 2.0 mmol), TBTU (0.64 g, 2.0 mmol), fluoroethylamine hydrochloride (0.40 g, 4.0 mmol) and triethylamine (1.62 g, 16.0 mmol) according to the general procedure F. A column chromatography (eluent EtOAc:methanol/10:1) yielded 0.25 g (35%) of base which was converted to hydrochloride salt by treatment with HCl.
Base; ¹H NMR (400 MHz, DMSO) δ 10.19 (s, 1H), 9.46 (d, *J*= 2.1 Hz, 1H), 8.70 (dd, *J* = 4.7, 1.4 Hz, 1H), 8.60 (dt, *J* = 8.0, 1.8 Hz, 1H), 8.52 (d, *J*= 5.8 Hz, 1H), 8.29 (dd, *J* = 9.5, 5.9 Hz, 1H), 7.93 (dd, *J* = 13 .9, 1.9 Hz, 1H), 7.72 (t,*J* = 8.6 Hz, 1H), 7.56 (dd, *J* = 8.0, 4.8 Hz, 1H), 7.51 (dd, *J* = 8.6,1.9 Hz, 1H), 6.85 (d, *J* = 5.8 Hz, 1H), 4.53 (dt, *J* = 47.5, 5.1 Hz, 2H), 3.56 (ddd, J= 25.8, 10.5, 5.1 Hz, 2H).
Salt; mp 194-196 °C (methanol); ¹H NMR (400 MHz, DMSO) δ 10.78 (bs, 1H), 9.49 (d, *J* = 1.7 Hz, 1H), 9.06 (d, *J* = 8.2 Hz, 1H), 8.95 (d, *J* = 4.2 Hz, 1H), 8.55 (d, *J* = 6.0 Hz, 1H), 8.32 (d, *J*= 3.7 Hz, 1H), 8.03 (dd, *J*= 8.1, 5.3 Hz, 1H), 7.82 (dd, *J* = 13.5, 1.7 Hz, 1H), 7.73 (t, *J* = 8.4 Hz, 1H), 7.64 (dd, *J*= 8.6, 1.9 Hz, 1H), 7.04 (d, *J*= 6.0 Hz, 1H), 4.53 (dt, *J* = 47.4, 5.4 Hz, 2H), 3.56 (ddd, J= 25.9, 10.6, 5.4 Hz, 2H).
¹³ C NMR (100 MHz, DMSO) δ 163.9, 161.6, 160.6, 159.5 (*J*_{C-F} = 246.7 Hz), 158.5, 154.6, 145.2, 143.3 (*J*_{C-F} = 11.7 Hz), 142.8 (*J*_{C-F} = 7.0 Hz), 13 5.2, 131.5, 127.3, 117.5 (*J*_{C-F} = 13.7 Hz), 115.9, 108.6, 107.2 (*J*_{C-F} = 28.1 Hz), 82.6 (*J*_{C-F} = 165.6 Hz), 40.5.
HR-MS (ESI) calcd. for C₁₈H₁₆F₂ON₅ [M+H⁺] 356.1317, found 356.1317.

### Example 48: 4-fluoro-N-(2-fluoroethyl)-3-((6-methyl-2-(pyrldin-3-yl)pyrimidin-4-yl)amino)benzamide hydrochloride (AMR-832)

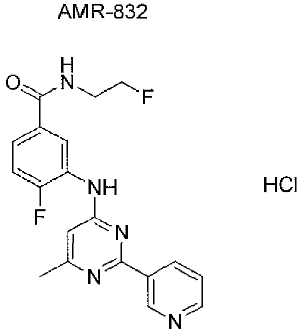

The title compound was prepared from 4-chloro-6-methyl-2-(pyridin-3-yl)-pyrimidine (0.33 g, 1.61 mmol), 3-amino-4-fluorobenzoic acid (0.25 g, 1.61 mmol), HBTU (0.61 g, 1.61 mmol), fluoroethylamine hydrochloride (0.32 g, 3.22 mmol) and triethylamine (1.30 g, 12.9 mmol) according to the general procedure F. A column chromatography (eluent EtOAc:methanol/7:1) yielded 0.37 g (62%) of base which was converted to hydrochloride salt by treatment with HCl. Base;¹H NMR (400 MHz, DMSO) δ 9.51 (s, 1H), 9.42 (d, *J*= 1.4 Hz, 1H), 8.83-8.68 (m, 2H), 8.65 (dd, *J*= 4.7, 1.7 Hz, 1H), 8.62-8.46 (m, 1H), 7.68 (ddd, *J* = 8.4, 4.6, 2.2 Hz, 1H), 7.51-7.45 (m, 1H), 7.41 (dd, *J* = 10.8, 8.6 Hz, 1H), 6.75 (s, 1H), 4.55 (dt, *J* = 47.4, 5.1 Hz, 2H), 3.58 (ddd, *J* = 26.3, 10.6, 5.3 Hz, 2H), 2.41 (s, 3H).
Salt; mp 194-199 °C (methanol); ¹H NMR (400 MHz, DMSO) δ 10.30 (bs, 1H), 9.39 (d, *J* = 1.8 Hz, 1H), 9.12-9.00 (m, 1H), 8.95 (dd, *J* = 5.4, 1.3 Hz, 1H), 8.92 (t, *J*= 5.5 Hz, 1H), 8.58 (d, *J* = 6.4 Hz, 1 H), 7.97 (dd, *J* = 8.0, 5.4 Hz, 1H), 7.81 (ddd, *J* = 8.3, 4.4, 2.2 Hz, 1H), 7.43 (dd, *J* = 10.4, 8.7 Hz, 1H), 6.91 (s, 1H), 4.57 (dt, *J* = 47.4, 5.2 Hz, 2H), 3.60 (ddd, *J* = 26.1, 10.4, 5.2 Hz, 2H), 2.48 (s, 3H).
¹⁹F NMR (376.5 MHz, DMSO) δ -221.6, -119.0.
¹³C NMR (100 MHz, DMSO) δ 165.3, 163.8, 161.2, 157.8, 156.5 (*J*_{C-F} = 251.2 Hz), 145.8, 143.4, 141.3, 134.0, 130.6, 126.2,125.9 (*J*_{C-F} = 12.0 Hz), 124.9, 115.8, 115.7, 104.9, 82.2 (*J*_{C-F} = 165.7 Hz), 40.0 (*J*_{C-F} = 21.1 Hz), 22.6.
HR-MS (ESI) calcd. for C₁₉H₁₈F₂ON₅ [M+H⁺] 370.1474, found 370.1473.

### Example 49: 1-(4-fluoro-3-((6-methyl-2-(pyridin-3-yl)pyrimidin-4-yl)amino)phenyl)ethanone hydrochloride (AMR-838)

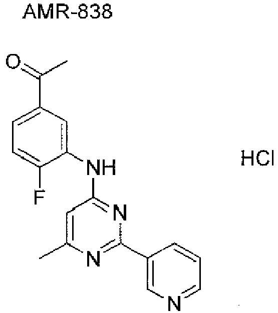

The title compound was prepared from 4-chloro-6-methyl-2-(pyridin-3-yl)-pyrimidine (0.33 g, 1.6 mmol), 3-amino-4-fluoroacetophenone (0.27 g, 1.77 mmol) and HCl (conc., 3 drops) according to the general procedure G. A column chromatography (eluent EtOAc:methanol/10:1) yielded 0.32 g (62%) of base which was converted to hydrochloride salt by treatment with HCl. Base; ¹H NMR (400 MHz, DMSO) δ 9.57 (s, 1H), 9.44 (dd, *J =* 2.2, 0.8 Hz, 1H), 8.91 (dd, *J =* 8.0, 2.2 Hz, 1H), 8.65 (dd, *J*= 4.8, 1.7 Hz, 1H), 8.63-8.49 (m, 1H), 7.77 (ddd, *J* = 8.5, 4.7, 2.2 Hz, 1H), 7.56-7.48 (m, 1H), 7.48-7.34 (m, 1H), 6.80 (s, 1H), 2.60 (s, 3H), 2.41 (s, 3H).
Salt; mp 193-194 °C (methanol); ¹H NMR (400 MHz, DMSO) δ 10.06 (s, 1H), 9.40 (s, 1H), 8.96 (d, *J*= 7.1 Hz, 1H), 8.89 (d, *J =* 5.1 Hz, 1H), 8.72 (d, *J* = 7.8 Hz, 1H), 8.00-7.87 (m, 1H), 7.87-7.66 (m, 1H), 7.47 (t, *J* = 9.6 Hz, 1H), 6.90 (s, 1H), 2.60 (s, 2H), 2.47 (s, 3H).
¹⁹FNMR (376.5 MHz, DMSO) δ-116.5.
¹³C NMR (100 MHz, DMSO) δ 196.9, 164.5, 161.4, 158.3, 156.5 (*J*_{C-F} = 253.6 Hz), 146.2, 143.7, 141.3, 134.6, 133.8, 127.1 (*J*_{C-F} = 12.1 Hz), 126.6, 126.3 (*J*_{C-F} = 8.5 Hz), 124.8, 116.5 (*J*_{C-F} = 20.6 Hz), 105.8, 27.2, 23.3.
HR-MS (ESI) calcd. for C₁₈H₁₆FON₄ [M+H⁺] 323.1303, found 323.1303.

### Example 50: 1-(2-fluoro-5-((6-methyl-2-(pyridin-3-yl)pyrimidin-4-yl)amino)phenyl)ethanone hydrochloride (AMR-839)

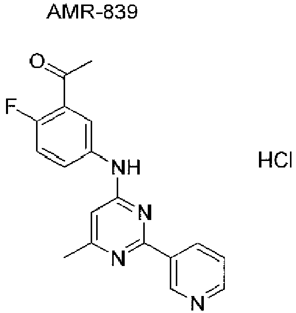

The title compound was prepared from 4-chloro-6-methyl-2-(pyridin-3-yl)-pyrimidine (0.33 g, 1.6 mmol), 5-amino-2-fluoroacetophenone (0.27 g, 1.77 mmol) and HCl (conc., 3 drops) according to the general procedure G. A column chromatography (eluent EtOAc:methanol /10:1) yielded 0.41 g (79%) of base which was converted to hydrochloride salt by treatment with HCl. Base; ¹H NMR (400 MHz, DMSO) δ 9.81 (s, 1H), 9.48 (dd, *J*= 2.1, 0.8 Hz, 1H), 8.74-8.51 (m, 2H), 8.41 (dd, *J*= 6.6, 2.9 Hz, 1H), 7.90 (ddd, *J* = 8.9, 4.2, 3.0 Hz, 1H), 7.51 (ddd, *J*= 8.0, 4.8, 0.8 Hz, 1H), 7.37 (dd, *J*= 10.8, 9.0 Hz, 1H), 6.58 (d, *J*= 0.6 Hz, 1H), 2.60 (d, *J* = 4.6 Hz, 3H), 2.40 (s, 3H).
Salt; mp > 200 °C (methanol); ¹H NMR (400 MHz, DMSO) δ 10.55 (s, 1H), 9.46 (d, *J* = 1.8 Hz, 1H), 8.98 (d, *J* = 8.1 Hz, 1H), 8.89 (dd, *J* = 5.2, 1.4 Hz, 1H), 8.38 (dd, *J =* 6.6, 2.8 Hz, 1H), 7.92 (ddd, *J*= 13.2, 7.8, 4.2 Hz, 2H), 7.37 (dd, *J*= 10.8, 9.0 Hz, 1H), 6.76 (s, 1H), 2.60 (d, *J*= 4.5 Hz, 3H), 2.45 (s, 3H).
¹⁹F NMR (376.5 MHz, DMSO) δ -116.5.
¹³C NMR (100 MHz, DMSO) δ195.3,163.4, 160.8, 158.5, 157.5 (*J*_{C-F} = 250.6 Hz), 147.2, 144.8, 140.7, 136.2, 134.0, 127.0 (*J*_{C-F} = 9.0 Hz), 126.1, 125.3 (*J*_{C-F} = 13.3 Hz), 121.4, 117.7 (*J*_{C-F} = 25.0 Hz), 105.6, 31.5 (*J*_{C-F} = 6.8 Hz), 22.9.
HR-MS (ESI) calcd. for C₁₈H₁₆FON₄ [M+H⁺] 323.1303, found 323.1301.

### III. Tests

The compounds synthesized were tested in ALPHA screen, ITC and for their ability to inhibit virus replication in TZM test.

### III.1: Tissue Culture Tests, TZM titration

For compounds testing the TZM cells were seeded on 96-well plate in the density 5 x 10³ cells per well. The culture media was aspirated approx. 16 hrs later and cells were infected by the wild type NL4.3 HIV-1 by addition of 50J..il of prepared virus stock diluted with 50 µl of complete DMEM. The compounds were added twice to ensure that there are not any preassembled viruses present at the time of their addition. Compounds were first added 8 hrs post-infection and then 24 hrs later. In both cases the old culture media were replaced by 100 µl of fresh complete DMEM prior to the compounds addition. Different compounds concentrations were tested, but the DMSO concentration never exceeded 1 % DMSO. As controls were used cells treated only with 1 % DMSO and cells treated with 0,1 µM Lopinavir (HIV-1 protease inhibitor). Supernatant was harvested 24 hrs after the second compound addition, i.e. 56 hrs post-infection. Harvested supernatants were immediately titrated on the fresh TZM cells in aim to perform the infectivity assay (TZM titration), or inactivated by addition of Triton X-100 to the final concentration 0.025 % for further ELISA analysis.

TZM titration was performed on the cells seeded day before on 96-well plate in the density 5 x 10³ cells per well. The analysis was performed for each compound concentration at three different dilutions and in duplicates. The culture media was aspired and cells were lysed 48 hrs post-infection by addition of 100 µl of SteadyGlo luciferase substrate (Promega) diluted 1 : 1 by complete DMEM. The luminescence signal was measured on Luminoskan Ascent (Thermo Labsystems) after the transfer of 80 µl of lysate to the flat bottom white polystyrene assay plates (Costar).

For ELISA measurement, the Maxisorp plates (Nunc) were precoated with monoclonal mouse anti HIV-1 CA antibodies at RT over night and blocked with 10 % FCS in PBS for at least 2 hrs at 37 °C prior the loading of inactivated supernatant. The analysis was done in three different dilutions and in duplicates for each compound concentration. For detection were used polyclonal rabbit anti HIV-1 CA antibodies and secondary antibodies labeled with horseradish peroxidase. The product of colorimetric reaction was quantified by measuring of absorbance at 450 mn at Multiscan EX reader (Thermo Scientific).

Cytotoxicity of selected hits tested in the assay with NL4.3 infected cells was determined by MTT test. TZM cells were seeded on the previous day on 96-well plate in the density 5 x 10³ cells per well. The tested compounds were added in different concentrations, but the DMSO concentration never exceeded 1 % DMSO. As controls were used cells treated only with 1 % DMSO (non-toxic concentration) and cells treated with 5 % DMSO (toxic concentration). 20 µl of the CellTiter 96 Aqueous One substrate (Promega) was added to the 100 µl of culture media 48 hrs after the compounds addition and the color development was quantified 4 hrs later by measuring the absorbance at 490 nm on Saphire reader. Compounds were consider non-cytotoxic (at given concentrations) when the measured values were higher than 80 % of the value of 1 % DMSO treated cells.

### III.2: Determination of the binding of tested compounds to the target :isothermal titration calorimetry (ITC)

The binding of CA ligand to the HIV capsid protein and its C-terminal part was monitared using a VP-ITC microcalorimeter (MicroCal Inc., Northampton, USA) at 25 °C. The solutions of reactants were prepared in 30 mM MES, pH 6.0 containing 10 % glycerol, 2 mM 2-mercaptoethanol, 2 % DMSO, and the exact concentrations of proteins were determined by amino acid analysis. Due to low solubility of some inhibitors, the precipitation was observed at concentration required for ITC measurement. The isothermal titration was used to estimate the ligand concentration and the binding parameters were calculated for situation when single inhibitor molecule binds to monomer or dimer of protein, respectively. Typically, 9 µl aliquots of pyrimidine derivatives were injected stepwise into the sample cell containing 1.43 ml of 7 µM protein until saturation was achieved. The experiment was accompanied by a corresponding control experiment in which inhibitor was injected into buffer alone. The association constants were determined by software developed by MicroCal.

### III.3: Alfa screening

The AlphaScreen experiments are performed using Perkin Elmer Enspire plate reader in white 96-well ProxiPlates and PBS supplemented with 0.05% Tween20, 2mM DTT, 2% DMSO. First, Streptavidin-coated Donor beads are used to capture the CAI-biotin peptide. In the other solution, a fusion protein of CA with glutathione-S-transferase (CA-GST) is mixed with GSH Acceptor beads. Both mixtures were incubated and then mixed together. To test the ability of prepared organic compounds to inhibit the CAI binding to CA, they are screened at concentrations between 0.5-100 µM and IC₅₀ values are calculated from the influenced signals.

### Principle of the assay

Upon illumination by laser at 680 nm, Donor beads containing the photosensitizer phthalocyanine convert ambient oxygen to an excited form of singlet oxygen ¹O₂. Singlet oxygen can within its half-life of ∼ 4µs diffuse approximately 200 nm in solution. If an Acceptor bead is within that proximity, which is mostly upon CA:CAI interaction, energy is transferred from the singlet oxygen to thioxene derivates within the Acceptor and signal at 520 - 620 nm is emitted. In the absence of an Acceptor bead, i.e. a compound inhibits CA/CAI interaction, singlet oxygen falls to ground state and no signal is produced.

**Table 4: Synthesized and tested compounds**

| **compound code** | **compound formula** | **Mr** | **α screen IC₅₀ (µM)** |
|---|---|---|---|
| KP-343 | 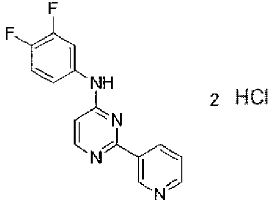 | 357 | 38 |
| KP-347 | 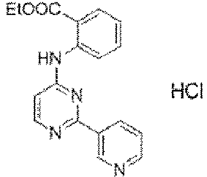 | 357 | > 100 |
| KP-351 | 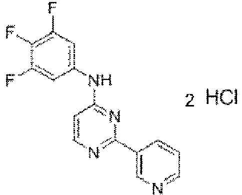 | 375 | 60 |
| KP-352 | 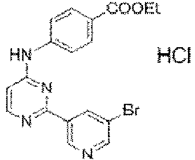 | 436 | > 100 |
| KP-355 | 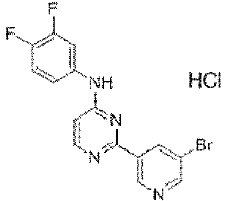 | 400 | > 100 |
| KP-359 | 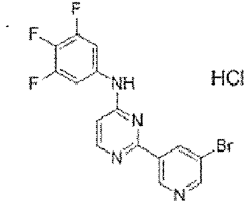 | 418 | > 100 |
| KP-360 | 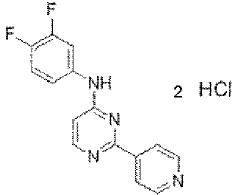 | 357 | 81 |
| KP-363 | 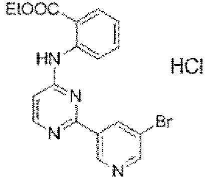 | 436 | 58 |
| KP-364 | 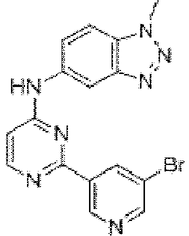 | 455 | 14 |
| KP-369 | 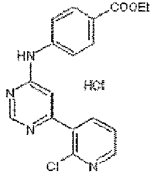 | 391 | > 100 |
| KP-373 | 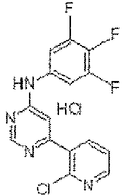 | 373 | > 100 |
| KP-374 | 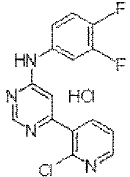 | 355 | > 100 |
| KP-375 | 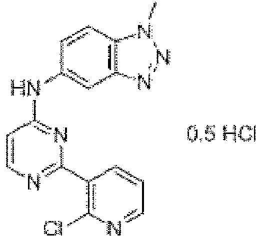 | 356 | > 100 |
| KP-377 | 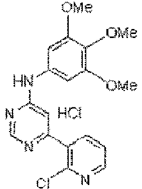 | 409 | > 100 |
| KP-380 | 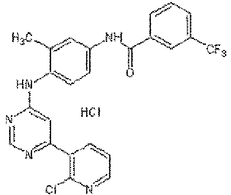 | 520 | > 100 |
| KP-384 | 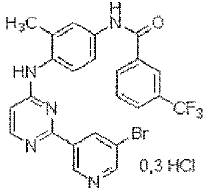 | 539 | > 100 |
| KP-385 | 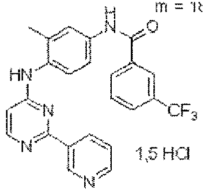 | 504 | > 100 |
| AMR-722 | 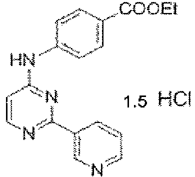 | 370 | 19 |
| AMR-723 | 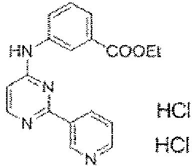 | 396 | 21 |
| AMR-725 | 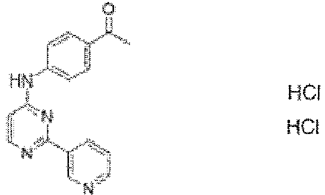 | 364 | 53 |
| AMR-726 | 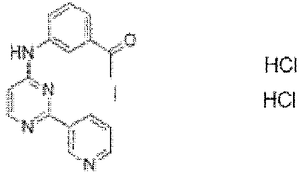 | 364 | 39 |
| AMR-742 | 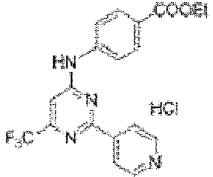 | 425 | > 100 |
| AMR-743 | 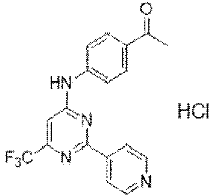 | 395 | > 100 |
| AMR-747 | 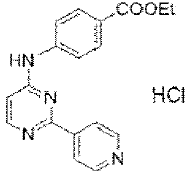 | 357 | 28 |
| AMR-748 | 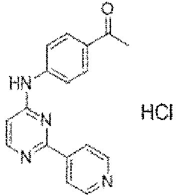 | 327 | > 100 |
| AMR-758 | 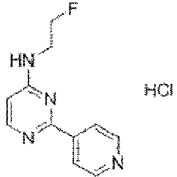 | 255 | > 100 |
| AMR-759 | 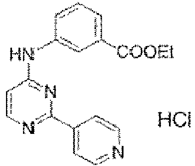 | 356 | > 100 |
| AMR-763 | 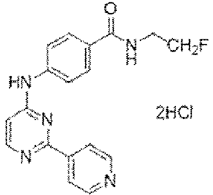 | 410 | > 100 |
| AMR-771 | 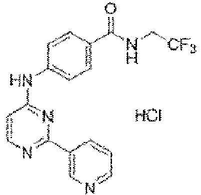 | 410 | 55 |
| AMR-778 | 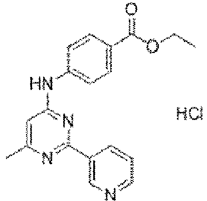 | 371 | 21 |
| AMR-792 | 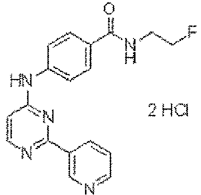 | 409 | 34 |
| AMR-794 | 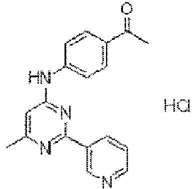 | 340 | 12 |
| AMR-795 | 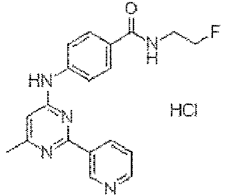 | 387 | 8 |
| AMR-802 | 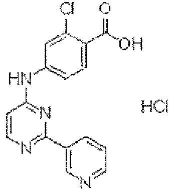 | 362 | 25 |
| AMR-808 | 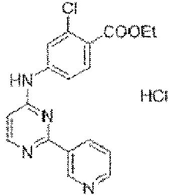 | 390 | 31 |
| AMR-816 | 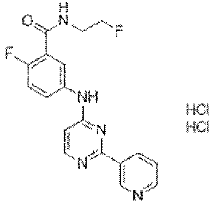 | 427 | 68 |
| AMR-822 | 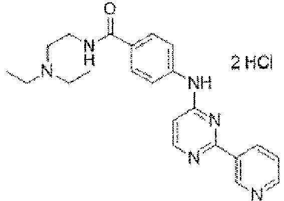 | 462 | > 100 |
| AMR-823 | 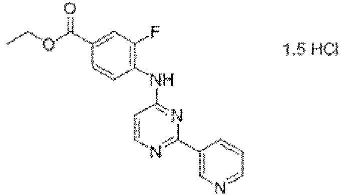 | 338 | 30 |
| AMR-825 | 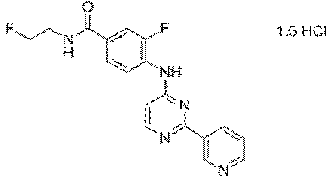 | 355 | 73 |
| AMR-827 | 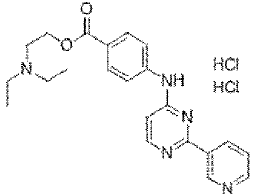 | 463 | > 100 |
| AMR-828 | 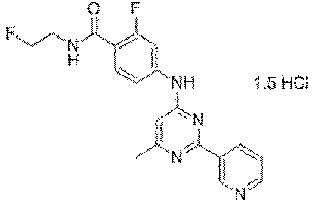 | 423 | 3 |
| AMR-829 | 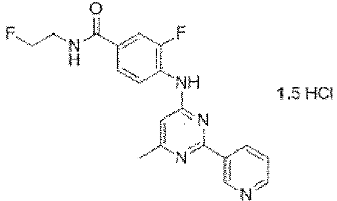 | 387 | 15 |
| AMR-830 | 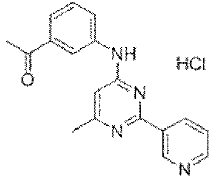 | 340 | 10 |
| AMR-831 | 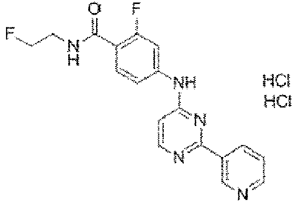 | 427 | 43 |
| AMR-832 | 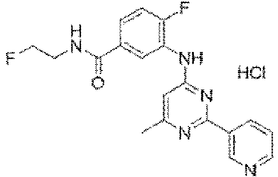 | 410 | 52 |
| AMR-838 | 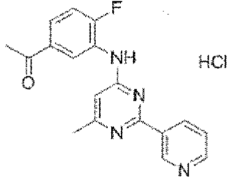 | 330 | > 100 |
| AMR-839 | 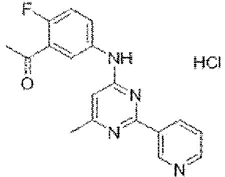 | 330 | 9 |

## Claims

1. A compound or a pharmaceutically acceptable salt or solvate thereof for use in inhibiting HIV capsid assembly, wherein the compound is a pyrimidine being at least substituted in two positions, preferably in 2 and 4 position or in 4 and 6 position, more preferably in 2 and 4 position.

2. The compound according to claim 1 having a structure according to formula (I) or (I*) 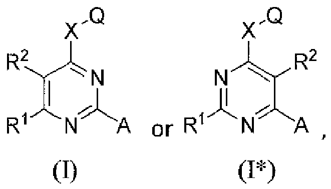 in particular according to formula (I) 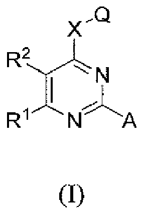 or a pharmaceutically acceptable salt or solvate thereof ,
wherein A is selected from the group consisting of aryl, heteroaryl and heterocycloalkyl group,
wherein X is selected from the group consisting of O, N(R³) and S, and wherein wherein R³ is selected from the group consisting of H, alkyl, cycloalkyl, aryl, heteroaryl and heterocycloalkyl,
wherein Q is selected from the group consisting of alkyl, cycloalkyl, aryl, heteroaryl and heterocycloalkyl,
and wherein R¹ and R², are, independently of each other, selected from the group consisting of H, D, -alkyl, cycloalkyl, cycloalkenyl, heterocycloalkyl, aryl, heteroaryl, alkenyl, alkoxy and halides.

3. The compound according to claim 2, wherein A is a, substituted or unsubstituted, heteroaryl group, preferably a, substituted or unsubstituted, heteroaryl group comprising at least one N, more preferably a, substituted or unsubstituted, pyridine group.

4. The compound according to claim 2 or 3 , wherein A has the structure 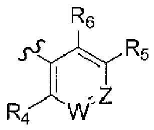 wherein W is N or C(R^{W}), and wherein Z is N or C(R^{Z}), and wherein R^{W} and R^{Z} are, independently of each other selected from the group consisting of H, alkyl, -O-alkyl, and halide,
and wherein R⁴, R⁵ and R⁶, are, independently of each other, selected from the group consisting of -H, -alkyl, -O-alkyl, -halides.

5. The compound according to claim 4, wherein A has the structure

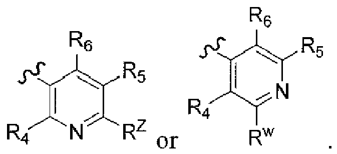

6. The compound according to claim 4 or 5, wherein R⁴, R⁵, R⁶, R^{W} and R^{Z} are, independently of each other selected from the group consisting of H, methyl, Br and Cl, preferably wherein A is selected from the group consisting of

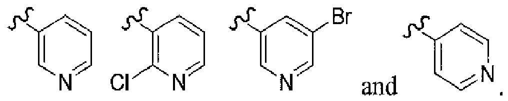

7. The compound according to any one of claims 2 to 5, wherein X is NH or N(CH₃), more preferably NH.

8. The compound according to any one of claims 2 to 6, wherein Q is 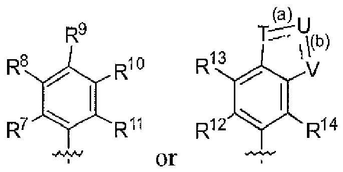 wherein R⁷, R⁸, R⁹, R¹⁰, R¹¹, R¹², R¹³ and R¹⁴ are, independently of each other, selected from the group consisting of -H, -alkyl, -halides, alkynyl, alkenyl, -NO₂, -CN, -C(=Y)-R¹⁵, -C(=Y)-H, -C(-Y)-Y¹-R¹⁵, -C(=Y)-OH,, -Y-R¹⁵, -NH₂, -NHR¹⁵,-NH -C(=Y)-R¹⁵, - CF₃, -S(O)R¹⁵, -SO₂R¹⁵, -P(O)R¹⁵R¹⁶, -P(O)(OR¹⁵)R¹⁶, -P(O)(OR¹⁵)(OR¹⁶), wherein Y is O, S or NH, and wherein Y¹ is O, S or NR*, wherein R* is H or alkyl, and wherein R¹⁵ and R¹⁶ are, independently of each other, selected from the group consisting of -H, - alkyl, -alkenyl, -heterocycloalkyl, aryl and heteroaryl,
and wherein
wherein T is -C-R^{T}, N or N-Y^{T}, with Y^{T} being H or alkyl
wherein U is -C-R^{U}, N or N-Y^{U}, with Y^{U} being H or alkyl
wherein V is -C-R^{V}, N or N-Y^{Y}, with Y^{V} being H or alkyl
the bond (a) is a single or double bond,
the bond (b) is a single or double bond,
with eth proviso that one of (a) and (b) is a double bond,
wherein R^{T}, R^{U}, and R^{V} are, independently of each other, H or -alkyl.

9. The compound according to claim 8, wherein Q is 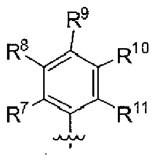 and wherein at least one of R⁷, R⁸, R⁹, R¹⁰ and R¹¹ is selected from the group consisting of -C(=O)-ethyl, -C(=O)-methyl -C(=O)-NH-CH₂-CH₂-F -C(=O)-H, -C(=O)-OH, -C(=O)-O-ethyl, -C(=O)-O-methyl -NH-C(=O)-OH, -O-methyl, -O-CH₂-CH₂-N(CH₂CH₃)₂, -NH-C(=Y)-(3-trifluoromethylphenyl) -CF₃, -Cl and -F.

10. The compound according to claim 8 or 9, wherein Q is selected from the group consisting

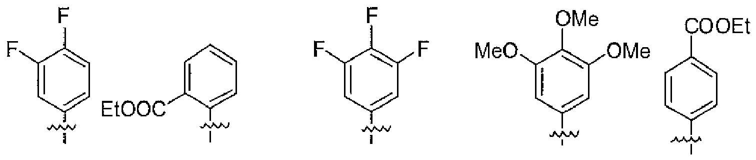

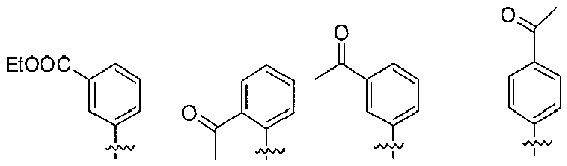

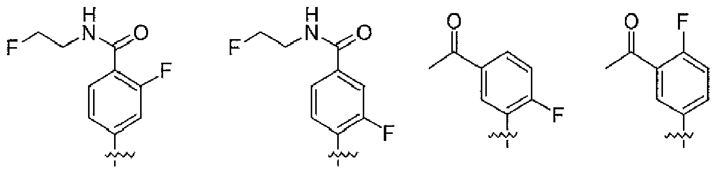

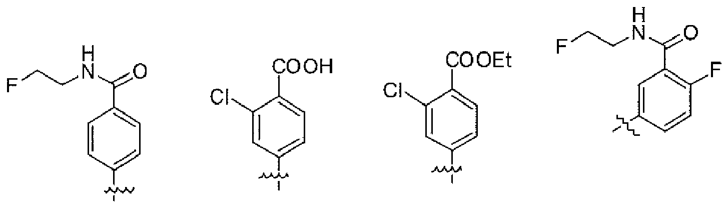

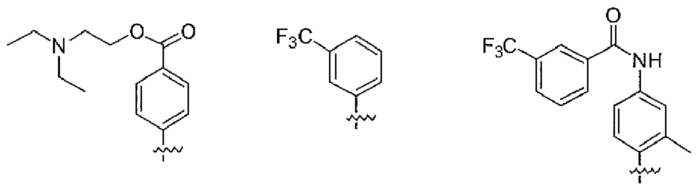

11. The compound according to claim 8, wherein Q is selected from the group consisting of

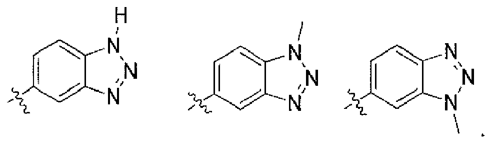

12. The compound according to claim 8, wherein Q is alkyl-F, preferably -CH₂-CH₂-F.

13. A compound according to any one of claims 1 to 12, wherein HIV is HIV-1 capsid assembly.

14. A pharmaceutical composition comprising a compound according to any one of claims 1 to 12.

15. Use of a compound as defined in any one of claims 1 to 12 for the manufacture of a medicament for inhibiting HIV capsid assembly.

16. A method of treating a patient suffering from HIV comprising administering a therapeutically effective amount of the compound as defined in any one of claims 1 to 12.
